# EUROPEAN PATENT SPECIFICATION

(11) **EP 2 224 820 B1**
(45) Date of publication and mention of the grant of the patent: **18.05.2011**
(21) Application number: 08838839.2
(22) Date of filing: 17.10.2008
(51) Int. Cl.: A23G 3/00, A23G 4/00, A61K 9/00

(54) **COMESTIBLE PRODUCTS**
ESSBARE PRDOUKTE
PRODUITS COMESTIBLES

(30) Priority: 18.10.2007 GB 0720516
(43) Date of publication of application: 08.09.2010
(73) Proprietor: Cadbury Holdings Limited, London W1J 6HB (GB)
(72) Inventor: PHILLIPS, Kerry, Louise, Wiltshire SN4 7TA (GB); BUSOLIN, Andre, F-79390 Thenezay (FR); MENANTEAU, Stephanie, F-86260 St. Pierre de Maille (FR); BENOIT, Yannick, F-78660 Prunay-En-Yvelines (FR); MOUSTAFA, Ousmane, F-59510 Hem (FR); LIEVEN, Jean-Michel, F-59263 Hauplin Ancoisne (FR); DEMEULEMEESTER, Patrice, F-59126 Linselles (FR); MONTAIGNE, Nathalie, F-59126 Linselles (FR); LAGACHE, Sylvie, F-59700 Marcq En Baroeul (FR)
(74) Representative: Wilson Gunn
(86) International application number: PCT/GB2008/003523
(87) International publication number: WO 2009/050479

(56) References cited:
- EP-A- 0 413 427
- EP-A- 0 613 684
- WO-A-01/28360
- GB-A- 1 125 383
- US-A- 4 983 378
- US-A1- 2007 031 561

## Description

The invention relates to comestible products containing galactose, to methods of preparing these products and their use. In particular, the invention relates to comestible products which induce a mouth-watering effect and to their use in the treatment of mouth dryness.

Many individuals suffer from dry-mouth, occasionally referred to as "xerostomia," from time to time due to a variety of physiological and environmental factors. Dry-mouth may be caused by reduced levels of saliva and can feel sticky and uncomfortable. Dry-mouth may lead to difficulties in tasting, chewing, swallowing and speaking, as well as a variety of more serious medical conditions. Prescription medications and artificial saliva are available for severe cases of dry-mouth. Individuals experiencing moderate levels of mouth dryness, however, often desire consumables that provide a sensation of hydration or mouth moistening. Although water is often sought for relief of mouth dryness, it is not always convenient or portable, and it does not provide long-lasting relief.

Compositions for the alleviation of dry-mouth are known, for instance WO 2007/022069 (Cadbury Adams USA LLC) describes a confectionary composition including a sweetening agent, a sweetness reducing agent, a food-grade acid, a flavour agent and optionally a cooling agent and/or a carrier. The sensation of mouth moistening is derived from the combination of reduced sweetness, the stimulation of salivation as a result of the sour taste from the acid and the sensation of cooling.

EP 0 413 427 (Warner-Lambert Company) describes a confectionary composition containing xylitol to stimulate salivation and hence address the problems of mouth dryness. US 4,983,378 (Parnell Pharmaceuticals, Inc.) also describes a composition which can alleviate dry-mouth, the composition comprising *Yerba Santa* fluid extract and a stimulator compound (such as an acid) to stimulate salivation.

There is a need, therefore, for comestibles, particularly confectioneries, which provide a sensation of mouth moistening upon consumption, which may be portable, such that they may be consumed whenever a feeling of mouth dryness is experienced by an individual, or merely to satisfy the taste preferences of the individual who wishes to consume a product with a "moist" sensation in the mouth. Further, there is a need for methods of preparing such comestibles and methods of treating xerostomia or other dry-mouth conditions by administering the same.

Accordingly, in a first aspect of the invention there is provided a comestible product according to claims 1-15.

Like glucose, galactose is a nutritive sweetener offering approximately 4 Kcal/g. Although being an optical isomer of glucose, galactose has only 60% of the sweetness of this compound and is classed as a low glycemic index (GI) sugar.

The use of galactose in food products is known, for instance, GB 1 125 383 (Walton John Smith) describes hard candy including galactose. In addition, GB 1 523 932 and GB 1 525 131 (both in the name of Tate & Lyle Limited) describe the use of galactose as a sweetener and as a sweetness modifier respectively. However, the use of galactose as a salivatory stimulator has not before been noted.

Therefore, according to a second aspect of the invention there is provided a method of improving the mouth-watering effect of a comestible comprising incorporating galactose into the comestible. The comestible may, in some embodiments additionally comprise a food-grade acid.

In a third aspect of the invention there is provided a method of preparing a comestible product in accordance with the first aspect comprising the incorporation of galactose into the comestible. Often, the incorporation will be selected from, coating the comestible with galactose, mixing galactose into the comestible and including one or more galactose containing zones in the comestible.

A fourth aspect of the invention provides the use of the comestible of the first aspect of the invention in the manufacture of a medicament for the treatment of xerostomia.

The invention offers a comestible product, and the use of a comestible product, which has mouth-watering properties but which does not exhibit a strong cooling sensation on the mouth and does not require the presence of food-grade acid, or acidic flavouring. This is achieved through the use of galactose as the primary sweetener in the comestible product. The galactose sweetened products exhibit a light refreshing sensation, and may be flavoured with flavouring ingredients not available where the sweetener offers a strong cooling sensation. This may be due to the masking of some more subtle flavours, or to unpalatabilility where the flavour is combined with a strong cooling effect. Similarly, as galactose does not have any substantial cooling effect in the mouth, it can be used to sweeten products where cooling would be inappropriate or undesirable.

A further benefit of the use of galactose as a sweetener is that it is possible to create a mouth-watering effect without the need to include high levels of food-grade acid or acidic flavouring (both known to induce salivation), and hence allows the production of products which alleviate xerostomia but which are not restricted to a sour taste experience. Berry (such as strawberry) and vanilla flavours are of particular interest.

In addition, it has been found that a galactose comestible is more salivating than a comparable sucrose comestible containing high levels of acid, and than a galactose comestible which also contains acid.

However, although the use of acid is not required to achieve the desirable mouth-watering effect of the comestible, as galactose is stable in the presence of acid, it is possible to include this, for instance, in embodiments where citrus (in particular lemon and/or lime) or mint flavours are to be used. The taste sensation derived from the combination of the galactose and acid has been found be particularly refreshing.

As galactose is metabolised more slowly than sucrose it is also advantageous to include galactose in the comestibles to provide a longer lasting source of energy than has previously been available. Studies by the applicant have shown that the inclusion of galactose in sports drinks is preferable to glucose as the incidence of hypoglycaemia is lower and the overall sporting performance is increased.

Additionally, the use of galactose in comestible products often allows the fat levels to be reduced. This is because galactose is less sweet than sucrose and so higher levels are required within a composition to provide the same sweetness. This allows some of the fat which would typically be present in a comestible to be substituted for galactose. Further, the low glycemic index of galactose (20) makes this sugar suitable for use in so-called "diabetic" foods. Although diabetic foods are known, these have typically used fructose or artificial sweeteners in substitute for glucose, and examples of this may be found in US 6,248,375 (Abbott Laboratories).

A further application for the use of galactose sweeteners is as a substitute for artificial sweeteners in "sugar free" products. Galactose sweeteners have the advantages that there is no laxative effect associated with galactose and that galactose can be classified as "natural", offering commercial opportunities in markets where the consumer is motivated to buy environmentally acceptable products.

In addition, it had generally been found that the higher the level of galactose in a product, the more distinct the mouth-watering sensation is. As used herein the expressions "mouth-watering", "salivating" and related expressions are used interchangeably and are intended to be construed as relating to the same effect.

### Comestibles

The comestible of the invention may be substantially any edible product, however it is preferred in this instance that the comestible be a foodstuff as opposed to a beverage. It is generally preferred that the comestible product of the invention have one or more features selected from a fat-content lower than the sucrose containing equivalent and a slower energy release than the sucrose containing equivalent.

It is often preferred that the food product be a confectionary product, often a candy product. Candy products are products which are generally primarily sugar based, for instance, chewy candy, hard boiled candy, jelly candy and other candies specific examples of which include caramel, toffee, fudge, praline, tablet, gumdrops, jelly beans, rock candy, lollipops, taffy, cotton candy, candy canes, peppermint sticks, peanut brittle, sucking candy, lozenges and candy bars. Additionally, the candy may be coated with a hard or soft shell and/or centre-filled. Further, the candy may be present in combination with another comestible, such as chewing gum.

The comestible may also be a chewing gum, which may be uncoated or coated with a hard or soft shell. Further, as with candy comestibles, the chewing gum may be centre-filled.

In embodiments where the comestible is a candy or chewing gum product, the comestible is preferably chewy. Chewy comestibles include chewing gum products, chewy candy and jelly candy. In many embodiments the chewy comestible will be chewy candy and/or chewing gum.

### Galactose

Galactose is the primary sweetener used in the comestible products of the invention. The use of the term "primary" is to be regarded as meaning the main sweetener, in that it is the sweetener present in the single highest amount. It has been found that it is necessary for galactose to be present as the primary sweetener if the mouth-watering effects of the invention are to be observed.

Some suitable levels of galactose are outlined below, however, the person skilled in the art would appreciate that other levels may be used to induce the mouth-watering effect of the invention and that the amount of galactose required in any one zone, or in the comestible as a whole, will very depending upon the composition and form of any particular comestible product.

The galactose levels may be measured as a percentage of the total sweetener in a composition. Galactose will be the primary sweetener in at least one zone of the comestible product. Preferably, galactose will be present in the at least one zone of the comestible in an amount greater than the total amount of the other sweeteners present in that zone. In some embodiments, galactose will be present in the comestible product an amount greater than the total amount of other sweeteners in the comestible. In some embodiments, the galactose level is preferably 30 or 40 - 100 wt% of the total sweetener in at least one zone of the comestible product, in some examples 45 - 98 wt%, often 60 - 95 wt%, more often 80 - 95 wt%, and in some embodiments 90 - 95 wt%. Alternatively, the galactose level may be 30 or 40-100 wt% of the total sweetener in the comestible product, in some examples 45 - 98 wt%, often 60 - 95 wt%, more often 80 - 95 wt%, and in some embodiments 90 - 95 wt%.

Galactose levels may also be measured as a percentage of the total composition. In some embodiments galactose will comprise 30-100 wt% of any galactose containing zone of the comestible, more specifically, from 40 - 95 wt%, in some examples from 55-90 wt%, often from 65 - 85 wt% of any zone to which galactose has been added. These preferred levels are currently believed to provide the optimal balance between product sweetness and the provision of the mouth-watering effect.

Where used in confectionary compositions, including candies and chewing gums, the galactose will typically be present in from 15-80 wt% of the total weight of any zone which comprises candy or gum; often from 25 - 60 wt%; in many embodiments from 30-55 wt% of the zone.

If the confectionary composition is a centre-fill composition, or includes an outer coating, galactose will typically be present in the centre-fill composition or the outer coating at a level of 30 - 100 wt% of the centre-fill or coating zone of the composition, in some examples 45 - 99 wt%, often 60 - 98 wt%, more often 80 - 95 wt%, and in some embodiments 90 - 95 wt% of the composition of the centre-fill or coating zone.

The term "substantially free from" is intended to mean that no galactose (or other component as appropriate) is added to the composition or zone. However, where a zone or comestible is substantially free from a particular ingredient, it is not intended that the presence of trace amounts of material be excluded, such as may arise from the use of modem production line techniques or through the presence of impurities in commercially available ingredients.

Although any commercially available galactose may be used in the compositions of the invention, it is preferred that the galactose be crystalline. In crystalline galactose the particle size will typically be up to in the region of 155 - 160 µm. In some embodiments, the galactose will be fine milled, often to less than 70 µm, preferably to less than 50 µm, in many embodiments to less than 30 µm as at this particle size the galactose particles cannot be detected on the tongue, removing any granular sensation in the mouth which may, in some products, be undesirable. Accordingly, the fine milled galactose may be present in the range 0 to one of 70, 50 or 30 µm. Further, the use of fine milled galactose in panned coatings is particularly desirable as the coatings produced have a silky texture in the mouth. As used herein, the term "particle size" and related terms describes the mean particle size of a component, this will be readily apparent to the person skilled in the art as any particulate matter will exhibit a particle size distribution range. This, however, may be characterised by the mean of the particle size distribution, as is the case here.

### Acid

Although the presence of a food-grade acid is not essential to the invention and the comestible may in some embodiments be free or substantially free from acid in one or more zones of the comestible product, there are embodiments where the comestible may desirably include a food-grade acid or salt thereof. Where the acid is absent, flavours may be included in the comestible which would be masked or possibly degraded by the presence of the acid (such as may sometimes be the case where berry or vanilla flavourings are used). However, where this is not a consideration, for instance where the comestible is citrus or mint flavoured, it may be preferred to include an acid in the comestible composition as these have been found to enhance the refreshing sensation provided by the invention.

The term "food-grade acid" is intended to include any acid which is acceptable for use in edible compositions. Such use implies a level of purity and acid strength outside which the acid would no longer be suitable for use in edible compositions, the determination of whether an acid constituted a food-grade acid would be familiar to the person skilled in the art. Typically, the acid will be selected from acids with a pKa in the range 1-5, such as malic acid, acetic acid, adipic acid, ascorbic acid, butyric acid, citric acid, formic acid, fumaric acid, glyconic acid, lactic acid, phosphoric acid, orthophosphoric acid, oxalic acid, succinic acid, tartaric acid, and combinations thereof. Commonly the acid will be selected from citric acid, malic acid, tartaric acid, orthophosphoric acid and combinations thereof. In some preferred embodiments from citric and/or malic acid.

The acid may be additionally or alternatively a salt of an acid. Where the comestible includes the salt of an acid it is preferably a salt of one of the acids listed above. Typically, the salt will be a group I or group II metal salt although ammonium and other organic salts are also envisaged. In many embodiments the acid salt will be a sodium or potassium salt, the most preferred salt for use in the invention being sodium citrate. It has been found that where the comestible includes a zone which is a hard coating a particularly refreshing coating may be obtained through the incorporation into the coating of acid salts. These can also in some instances have a crystallisation retarding effect as described below.

In embodiments where it is preferred that the food-grade acid or salt thereof be absent, it is preferably at a level of 0.1 wt% or less in at least one zone of the comestible. Alternatively the acid or acid salt may be present at a level of 0.1 wt% or less of the entire comestible product. In such embodiments it is preferred that at least one zone of the comestible be substantially free of acid or acid salt, or alternatively that the comestible product be substantially free of this component.

Where present as an optional component, the acid may be present in the range 0.1 - 85 wt% of any zone containing the acid, preferably from 0.25 - 20 wt%, often from 0.5-10 wt% of any acid containing zone.

### Flavourings

The comestible products also preferably contain flavours (i.e., flavourings or flavour agents). Flavours which may be used include those flavours known to the skilled artisan, such as natural and artificial flavours. These flavours may be chosen from synthetic flavour oils and flavouring aromatics and/or oils, oleoresins and extracts derived from plants, leaves, flowers, fruits, and so forth, and combinations thereof. Non-limiting representative flavour oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavourings are artificial, natural and synthetic fruit flavours such as vanilla, and citrus oils including lemon, orange, lime, grapefruit, yazu, sudachi, and fruit essences including apple, pear, peach, grape, strawberry, raspberry, cherry, plum, pineapple, watermelon, apricot, banana, melon, ume, cherry, raspberry, blackberry, tropical fruit, mango, mangosteen, pomegranate, papaya, and so forth. Other potential flavours include a milk flavour, a butter flavour, a cheese flavour, a cream flavour, and a yogurt flavour; a vanilla flavour; tea or coffee flavours, such as a green tea flavour, a oolong tea flavour, a tea flavour, a cocoa flavour, a chocolate flavour, and a coffee flavour; mint flavours, such as a peppermint flavour, a spearmint flavour, and a Japanese mint flavour; spicy flavours, such as an asafetida flavour, an ajowan flavour, an anise flavour, an angelica flavour, a fennel flavour, an allspice flavour, a cinnamon flavour, a camomile flavour, a mustard flavour, a cardamom flavour, a caraway flavour, a cumin flavour, a clove flavour, a pepper flavour, a coriander flavour, a sassafras flavour, a savoury flavour, a Zanthoxyli Fructus flavour, a perilla flavour, a juniper berry flavour, a ginger flavour, a star anise flavour, a horseradish flavour, a thyme flavour, a tarragon flavour, a dill flavour, a capsicum flavour, a nutmeg flavour, a basil flavour, a marjoram flavour, a rosemary flavour, a bayleaf flavour, and a wasabi (Japanese horseradish) flavour; alcoholic flavours, such as a wine flavour, a whisky flavour, a brandy flavour, a rum flavour, a gin flavour, and a liqueur flavour; floral flavours; and vegetable flavours, such as an onion flavour, a garlic flavour, a cabbage flavour, a carrot flavour, a celery flavour, mushroom flavour, and a tomato flavour. These flavours may be used in liquid or solid form and may be used individually or in combination.

Other useful flavourings include aldehydes and esters such as cinnamyl acetate, cinnamaldehyde, citral diethylacetal, dihydrocarvyl acetate, eugenyl formate, p-methylamisol, and so forth may be used. Generally any flavouring or food additive such as those described in Chemicals Used in Food Processing, publication 1274, pages 63-258, by the National Academy of Sciences, may be used.

Further examples of aldehyde flavourings include but are not limited to acetaldehyde (apple), benzaldehyde (cherry, almond), anisic aldehyde (liquorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavours), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, i.e., melonal (melon), 2,6-dimethyloctanal (green fruit), and 2-dodecenal (citrus, mandarin), cherry, grape, strawberry shortcake, and mixtures thereof.

Preferred flavourings include citrus flavours, in particular, lemon and lime flavours; berry flavours, such as strawberry; mint flavours and vanilla flavour. Citrus flavours are preferred where the comestible, or the zone or zones of the comestible in which the flavour will be present, includes a food-grade acid as the combination of the food-grade acid and a citrus or mint flavour has been found to be particularly refreshing. Further, the inclusion of galactose in that zone or another zone of the comestible with its salivatory effects further enhances the refreshing sensation derived from the combination of a citrus flavour and food-grade acid.

In some embodiments, the flavour agent may be employed in either liquid form and/or dried form. When employed in the latter form, suitable drying means such as spray drying the oil may be used. Alternatively, the flavour agent may be absorbed onto water-soluble materials, such as cellulose, starch, sugar, maltodextrin, gum arabic and so forth or may be encapsulated. The actual techniques for preparing such dried forms are well-known.

In some embodiments, the flavour agents may be used in many distinct physical forms well-known in the art to provide an initial burst of flavour and/or a prolonged sensation of flavour. Without being limited thereto, such physical forms include free forms, such as spray dried, powdered, beaded forms, encapsulated forms, and mixtures thereof.

The flavours of the invention may be used alone or in combination. Further, when used in combination they may be combined within a single zone or in combination as single flavours in different zones of a multi-zone comestible.

The amount of flavour agent employed herein may be a matter of preference subject to such factors as the individual flavour and the strength of flavour desired. Thus, the amount of flavouring may be varied in order to obtain the result desired in the final product and such variations are within the capabilities of those skilled in the art without the need for undue experimentation. In general, the flavour agent is present in amounts from 0.001 - 5.0 wt% and, more specifically, from 0.05 - 4.0 wt% of the flavour containing zone, and even more specifically, 0.1 - 3.0 wt%.

### Multi-zone Products

The comestible product of the invention contains at least one zone. The term "zone" is intended to mean an area or region which is distinguished from adjacent parts by a distinctive feature or characteristic. Accordingly, within any zone, the properties and composition of the comestible will often be substantially uniform.

As noted above, galactose will be present in one or more zones of the comestibles of the invention. Some comestibles may comprise a single zone only, such as lozenges or cotton candy; other comestible products may contain more than one zone. There may be any number of zones, however, preferably the number of zones will be in the range 1-100. More often, the number of zones will be in the range 1-10, typically, 1 - 5 in some embodiments 1-3. For instance, some confectionary products may have 1, 2 or 3 zones, selected from a centre-fill region, a candy or chewing-gum core and an outer coating. The confectionary may comprise, for example, a candy/gum core only (1 zone), a core and a centre-fill (2 zones), a core and an outer coating (2 zones), or a centre-fill, core and outer coating (3 zones).

Where the comestible product is a candy or chewing gum product comprising 2 or 3 zones, wherein the zones are a core, a second zone selected from a centre-fill and an outer coating and optionally a third zone selected from the other of the centre-fill and outer coating, it will sometimes be the case that galactose is the primary sweetener in the second zone. Optionally, galactose may also be the primary sweetener in the third zone. In other embodiments, galactose may also or alternatively be the primary sweetener in the first zone, the core.

It will often be the case that galactose is the primary sweetener in more than one zone of the comestible product. However, in some embodiments it may be desired, for flexibility of taste sensation, that there is at least one zone which does not contain galactose as the primary sweetener. Further, there may be at least one zone which contains galactose in the range 0 - 0.5 wt%, or less than 0.5 wt% (by weight of the zone) or which is substantially free from galactose.

### Centre-fill Products

Where the comestible of the invention includes a centre-fill zone, the centre-fill zone may be a solid composition, a liquid composition or gaseous. Solid compositions include those which effervesce upon contact with water. The effervescing composition will typically comprise galactose and optionally a food-grade acid, such as is described above. In some embodiments, a secondary sweetener may be also included, this may be a bulk sweetener and/or an artificial sweetener. Flavourings and colourings may also be present.

In some embodiments, the centre-fill zone may be substantially or completely filled with the liquid, solid, semi-solid or gaseous centre-fill composition. In some other embodiments, the centre-fill region may be only partially filled with the liquid, solid, semi-solid or gaseous centre-fill composition.

The centre-fill zone may include two or more centre-fill compositions, these may be in the same or different forms. For example, the centre-fill composition may comprise two or more immiscible liquids; two or more distinct solids, semi-solids or gases; combinations of liquids and solids; and combinations of two or more liquids, solids, semi-solids and/or gases. These may be present in the same or different amounts and may have similar or distinct characteristics of, for example, viscosity, colour, flavour, taste, sensation, ingredient components, functional components, or the like. The solid components of the centre-fill composition may be flavour beads, fruit particles, nut particles, flavour particles, gelatine portions, and the like.

Additionally in some embodiments, the physical form of the centre-fill zone can change. For instance, the centre can be solid when manufactured and then become liquid over time. This could be through enzymic action upon the centre-fill composition or due to manufacture at a temperature below the melting point of the centre-fill composition and storage at a temperature above the melting point temperature.

The centre-fill further may have total moisture content including free and bound moisture from 0-35 wt% of said centre-fill. As used herein, the term "moisture" is intended to include all liquid solvents, however, in the majority of embodiments the only moisture present will be derived from the presence of water.

### LIQUID CENTRE

A liquid centre will typically be aqueous, although non-aqueous embodiments are contemplated. Solutions, suspensions and emulsions may be used, more often solutions. Often the viscosity of the liquid centre will be in the range 300 to 10,000 cp ad 25°C, preferably in the range 4,000 to 6,500 cp, for ease of processing. To improve microbial stability and provide a pleasurable texture, it is generally preferred that the water activity of the liquid be in the range 0.1 to 0.7, often in the range 0.25 to 0.35.

The liquid centre may comprise, for instance, fruit juice; vegetable juice; fruit puree; fruit pulp; vegetable pulp; vegetable puree; fruit sauce; vegetable sauce; honey; maple syrup; molasses; corn syrup; sugar syrup; polyol syrup; hydrogenated starch hydrolysates syrup; emulsions; vegetable oil; glycerin; propylene glycol; ethanol; liqueurs; chocolate syrup, dairy-based liquids such as milk, cream, etc.; and combinations thereof. However, where the centre contains a sweet component, this will be in addition to galactose and preferably in a lower percentage of the total liquid composition.

The composition may also include a natural or synthetic gum such as carboxymethylcellulose, pectin, propylene glycol aginate, agar and gum tragacanth. The gums serve to increase viscosity by reducing the amount of free water in the composition. Where a gum is present, the viscosity of the centre-fill may be in the range 3,000 cp to about 6,000 cp at 25 °C. Xanthan gum may also be used to increase the viscosity of the centre-fill composition. Increasing the viscosity of the liquid may help to prevent the centre-fill composition from leaking through the comestible.

### SOLID CENTRE

The solid centre can include particulates including, but not limited to nuts; seeds; cocoa beans; coffee beans; milk powders; fruit-containing particles; freeze dried fruit; freeze dried vegetables; fat particles; cocoa powder; sucrose; starch; polyols such as sorbitol, mannitol, maltitol, isomalt, hydrogenated starch hydrolysates; waxes; and combinations thereof. The particulates may be substances onto which other materials have been absorbed.

The solid centre can also be substantially unitary in composition and may comprise chocolate, compound coating, carob coating, cocoa butter, butter fat, hydrogenated vegetable fat, illipe butter, fondant including fondant-based cremes, fudge, frappe, caramel, nougat, compressed tablet, cotton candy, marzipan, hard boiled candy, gummy candy, jelly beans, toffees, jellies including pectin-based gels, jams, preserves, butterscotch, nut brittles or croquant, candied fruit, marshmallow, pastilles, pralines or nougats, flour or starch confectionary, truffles, nonpareils, bon bons, afterdinner mints, fourres, nut pastes, peanut butter, chewing gum, kisses, angel kisses, montelimart, nougatine, fruit chews, Turkish delight, hard gums, soft gums, starch jellies, gelatin jellies, agar jellies, persipan, coconut paste, coconut ice, lozenges, cachous, creme paste, dragees, sugared nuts, sugared almonds, comfits, aniseed balls, liquorice, liquorice paste, chocolate spreads, chocolate crumb, and combinations thereof. In one possible embodiment, the centre-fill composition is effervescent and comprises, for instance, an acid and a base which react together when moistened in the mouth to produce a "fizz". In such an embodiment the acid and base may each independently comprise up to 20 wt% of the centre-fill composition, more typically up to 15 wt%, preferably from 5-15 wt% and often approximately 10 wt%. It is preferred that the acid and base are both present in substantially equal amounts. In many effervescent embodiments, the sole additional component is galactose.

### GASEOUS CENTRE

Gaseous centres can form a void in the comestible composition that alters the comestible composition's texture profile by collapsing upon chewing. The gaseous centre can include a single trapped gas such as nitrogen, oxygen or carbon dioxide while in other embodiments, the gaseous centre can include a mixed gas composition such as air. In some embodiments, the gas can be included in the centre as part of a matrix such as a foam or glassy matrix. In embodiments where the gas can be trapped in a glassy candy matrix, the glass matrix will typically be galactose and the gas carbon dioxide. Where the centre is a foam, the foam will often include milk proteins and the gas a mixed gas, such as air.

Any of the centre-fill compositions discussed above may include any components known in the art for incorporation with a centre-fill composition, for instance, as described elsewhere in this application. Most centre-fill confectionary comprises from 5-20 wt% of the product of centre-fill composition, in some embodiments 5 - 15 wt%, more often from 7-12 wt%.

### Coated Products

The outer coating may be hard, crunchy, or soft. The coating composition may range from 2-80 wt%, often 20 - 40 wt%, in some embodiments 25-35 wt% of the comestible. The coating of the invention will typically contain galactose as the principle sweetener, often as the principle component, however, it may be desired that the coating have different properties to the core and accordingly the coating may comprise sugar or a polyol as would be known in the art. In addition, the coating may include flavours, colours, etc. and may be crystalline or amorphous.

The outer coating may include, in addition to galactose, sorbitol, maltitol, isomalt, other crystallisable polyols and/or sucrose as a minor ingredient. Alternatively, where the coating is intended to offer a sweet outer layer which, for instance, releases a mouth-watering centre, the outer coating may include galactose as a minor ingredient, or not at all.

The coating may be total or partial in the sense that it may entirely encapsulate the comestible, or cover only a defined area. For instance, a dual textured and aesthetically pleasing product could be provided by coating only one half of the comestible. Further, the coating may contain many or few layers as would be apparent to the person skilled in the art. For instance, a thin coating could be provided to offer an initial crunch, but quick dissolution in the oral cavity. Alternatively, a thick coating could be provided, for example, in instances where the comestible was particularly easily deformable and required protection during transport and storage.

The coating may include several opaque layers, such that the coated composition is not visible through the coating itself, which can optionally be covered with a further one or more transparent layers for aesthetic, textural and protective purposes. The outer coating may also contain small amounts of water and gum arabic. The coating can be further coated with wax to improve the appearance of the comestible, however, the presence of a waxy coating delays the onset of the mouth-watering sensation offered by the inventive compositions. The coating may be applied in a conventional manner by successive applications of a coating solution, with drying in between each coat. As the coating dries it usually becomes opaque and is usually white, though other colourants may be added.

Coatings which are primarily composed of galactose require new panning techniques as a result of the difference in physical properties between galactose coatings and the known sucrose, glucose and/or sugar-free coatings. In particular, the use of known hard and soft panning techniques is undesirable as galactose crystallises slowly, resulting in a long drying time.

However, the coating obtained is of good quality, has a nice crispy texture and a mouth-watering effect. Further, the surface is smooth and has a "silky" feel in the mouth. In addition, it has been possible to develop coatings with an appearance reminiscent to that of a lemon skin, wherein the outer surface of the coating appears cracked but remains smooth to the touch. Whilst not wishing to be bound by theory, this is thought to be as a result of moisture migration through the coating from coating syrup to comestible core.

The galactose coating may be used with any confectionary core, however it has been found to work particularly well in combination with chewing gum cores where the natural moisture barrier of the centre results in a particularly smooth coating surface.

Where a galactose coating is to be applied the comestible core will typically be pre-coated to provide a surface onto which the outer coating can adhere and to smooth the surface of the candy. Whilst the pre-coating is optional, it is generally preferred as the quality of the final coating is improved where a pre-coating is present. In some embodiments, there will be two or more pre-coating layers interspersed with coating layers before the outer coating is applied. This has been found to provide a more effective covering than the use of multiple pre-coating layers and then the application of the outer coating to these.

The pre-coating may include an application of polyvinyl acetate (PVA). This may be applied as a solution of PVA in a solvent, such as ethyl alcohol. When an outer hard coating is desired, the PVA application may be approximately 3-4 wt% of the total coating or about 1 wt% of the total weight of the comestible. Alternatively, the pre-coating may be a dilution of the outer coating composition. For instance, the pre-coating may comprise from 1-99 wt% outer coating composition with the remaining 99 - 1 wt% of the composition being solvent. Preferably the outer coating composition will comprise 1-50 wt%, often 1-25 wt%, in some instances 1-15 wt% of the pre-coating; solvent comprising the other component of the pre-coating. Typically the solvent will be water, although other solvents, in particular ethanol, are also envisaged.

Whilst short drying times are desirable for economic reasons, it has been found that if crystallisation of the galactose occurs too quickly the surface of the coating is uneven and lacking in aesthetic appeal. In some embodiments, crystallisation may be slowed using a crystallisation retarding component selected from a gum, polydextrose, sodium citrate, gelatine, mannitol and/or combinations thereof. By "crystallisation retarding component" we mean any compound which is capable of reducing the rate of crystallisation of the galactose in the coating composition.

Where a gum is used to slow down crystallisation, this may be selected from, for instance, gum Arabic, gum talha, xanthan gum, carrageenan and/or mixtures thereof. Of the gums, gum Arabic is preferred. Where gums are used, these will typically be present in the range 0.5 - 15 wt%, often 1-10 wt%, most often 2-6 wt% of the coating. The presence of high levels of gum in the coating composition has been found to enhance the crispiness of the coating.

Alternatively or in addition to the use of gums, polydextrose and/or mannitol can be utilised to slow crystallisation, where used, the polydextrose and/or mannitol are preferably at a level in the range 5-30 wt%, in some embodiments 10-20 wt% of the coating. Of the possible crystallisation retarding components, the use of polydextrose is particularly preferred due to ease of processing the coating composition and the excellent crispiness of the coating obtained. Often the polydextrose will be used at a level of approximately 15 wt% of the coating composition. Sodium citrate will typically be used at levels in the range 0.5-15 wt%, often 1-10 wt%, most often 2-6 wt% of the coating. However, where gelatine is used, it will typically be included in the composition at levels in the range 0.1-10 wt%, in some embodiments 0.5 - 5 wt%, more specifically 1-4 wt% of the coating.

In addition, it has been found that by controlling the pH of the water used as part of the coating, the colour of the coating can be controlled, for instance, using distilled water provides a clear solution, whereas water at other pH's can lead to a yellowing of the coating once applied. Further, it has been found that surface crystallisation of the galactose solution can be inhibited by panning the comestible in a substantially closed system.

The coating can further include coloured flakes or speckles. Cellulosics such as carboxymethyl cellulose, pullulan, alginate, starch, polyvinyl acetate or combinations thereof can also be dispersed throughout the coating.

As with the general disclosure of the invention, it is preferred that the galactose used in the coatings be crystalline, often fine milled to less than 70 µm, in some examples to less than 50 µm and in many embodiments to less than 30 µm as at this particle size a particularly smooth shell may be obtained.

### Optional Components

The comestible of the invention may include a variety of optional components as would be familiar to one skilled in the art. Examples of these optional components are listed below. For multi-zone products the optional components may be present in one or more of the zones as appropriate for the function to be served. Further, the optional components may be used alone or in combination whether that combination is within a single zone of a multi-zone product or in different zones within the comestible.

### Sweeteners

The comestible of the invention may additionally contain at least one sweetener other than galactose. As used herein, the term "sweetener" should be taken to include any component of the comestible composition which imparts sweetness, in particular, the sweeteners listed below. Where galactose is present in a zone as the primary sweetener, each additional sweetener will be present in an amount less than the galactose. In many embodiments the total amount of additional sweetener will be less than the amount of galactose present in the zone. The additional sweeteners may be any known bulk or intense sweetener.

### BULK SWEETENER

Examples of suitable bulk sweeteners include saccharides, sugarless bulk sweeteners, or the like, or mixtures thereof. Saccharides, or sugars, are known in the art for providing sweet taste sensations for a consumer, thereby providing the sensation of sweetness to a foodstuff or composition to which they are added. Saccharides are based upon relatively simple carbohydrates comprising one or more monosaccharide units. Suitable saccharides for the present invention include, by way of example, those selected from the groups comprising monosaccharides, disaccharides, and polysaccharides.

Suitable monosaccharides may be selected from Trioses - for example glyceraldehydes, or dihydroxyacetone; tetroses - for example erythrose, threose, or erythrulose; pentoses - for example arabinose, lyxose, ribose, xylose, ribulose, or xylulose; hexoses - for example allose, altrose, glucose, idose, mannose, talose, fructose, psicose, sorbose, or tagatose; heptoses - for example mannoheptulose, or sedoheptulose; octoses - for example octolose, or 2-keto-3-deoxy-manno-octonate; and nonoses - for example sialose. Suitable disaccharides may be selected from, for example, sucrose, lactose, maltose, trehalose, or cellobiose. Suitable polysaccharides include starch and glycogen. Preferred saccharides are those selected from monosaccharides, and in particular those selected from the hexoses group. Particularly preferred monosaccharides are glucose and fructose.

Sugar alcohols (also known as polyols) may also be present in the comestible of the invention. Sugar alcohols are a hydrogenated or partially hydrogenated form of carbohydrate in which the carbonyl group (aldehyde or ketone) is reduced to a primary or secondary hydroxyl group. Suitable sugar alcohols may include those derived from disaccharides or monosaccharides and may include erythritol, isomalt, lactitol, galactitol, hydrogenated starch hydrolysates, polyglycitol syrups, maltitol, polydextrose, mannitol, sorbitol, xylitol, or any combination thereof. Particularly preferred sugar alcohols include maltitol, sorbitol and polydextrose.

Specific examples of suitable alcohol sugars include, by way of example, C* Eridex (available commercially from Cargill of Mechelen, Belgium), Neosorb P60, Maltisorb P90, Xylisorb 90, Mannitol 60 (all available commercially from Roquette of Lestrem, France), Isomalt PF (available commercially from Palatinit of Germany), and Lactitol (available commercially from Danisco of Copenhagen, Denmark).

The bulk sweetener may be present alone or in any suitable combination in the range 0.01 - 49 wt% total sweetener. More preferably, the bulk sweetener is present in the range 5-45 wt%. Most preferably, in the range 5-20 wt%. When the sole secondary sweetener is a bulk sweetener, this sweetener will often be present in the range 25 - 49 wt%, in some embodiments 30-45 wt% of the total sweetener.

### INTENSE SWEETENER

Without being limited to particular sweeteners, representative categories and examples of intense sweeteners include: a) water-soluble sweetening agents such as dihydrochalcones, monellin, stevia, steviosides, rebaudioside A, glycyrrhizin, dihydroflavenol, and L-aminodicarboxylic acid aminoalkenoic acid ester amides, such as those disclosed in U.S. Pat. No. 4,619,834, and mixtures thereof; b) water-soluble artificial sweeteners such as soluble saccharin salts, i.e., sodium or calcium saccharin salts, cyclamate salts, the sodium, ammonium or calcium salt of 3,4- dihydro-6-methyl-1,2,3-oxathiazine-4-one-2,2-dioxide, the potassium salt of 3,4-dihydro-6- methyl-1,2,3-oxathiazine-4-one-2,2-dioxide (Acesulfame-K), the free acid form of saccharin, and mixtures thereof; c) dipeptide based sweeteners, such as L-aspartic acid derived sweeteners, such as L-aspartyl-L-phenylalanine methyl ester (Aspartame) and materials described in U.S. Pat. No. 3,492,131, L-alphaaspartyl-N-(2,2,4,4-tetramethyl-3-thietanyl)-D-alaninamide hydrate (Alitame), N-[N-(3,3-dimethylbutyl)-L-aspartyl]-L-phenylalanine 1-methyl ester (Neotame), methyl esters of L-aspartyl-L-phenylglycerine and L-aspartyl-L-2,5-dihydrophenyl-glycine, L-aspartyl-2,5-dihydro-L-phenylalanine; L-aspartyl~L-(1-cyclohexen)-alanine, and mixtures thereof; d) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha- D-fructofuranoside, or 4-chloro-4-deoxygalactosucrose; 4-chloro-4-deoxy-alpha-D- galactopyranosyl- 1 -chloro-1-deoxy-beta-D-fructo-furanoside, or 4, 1 '-dichloro-4, 1 '- dideoxygalactosucrose; r,6'-dichlorol,6'-dideoxysucrose; 4-chloro-4-deoxy-alpha-D-galactopyranosyl-1-dichloro-o-dideoxy-beta-D-fructo furanoside, or 4,1,6'-trichloro- 4,1',6'-trideoxygalactosucrose; 4,6-dichloro-4,6-dideoxy-alpha-D-galactopyranosyl-6-chloro- 6-deoxy-beta-D- fructofuranoside, or 4,6,6'-trichloro-4,6,6'-trideoxygalactosucrose; 6,1',6'- trichloro-6,r,6'-trideoxysucrose; 4,6-dichloro-4,6-dideoxy-alpha-D-galacto-pyranosyl-1,6- dichloro-1,6-dideox y-beta-D-fructofuranoside, or 4,6,1',6'-tetrachloro4,6,r,6'- tetradeoxygalacto-sucrose; and 4,6,1,6'-tetradeoxy-sucrose, and mixtures thereof; e) protein based sweeteners such as thaumatococcus danielli (Thaumatin I and II) and talin; f) the sweetener monatin (2-hydroxy-2-(indol-3-ylmethyl)-4-aminoglutaric acid) and its derivatives; and g) the sweetener Lo han guo (sometimes also referred to as "Lo han kuo").

Intense sweetening agents may be used in many distinct physical forms well-known in the art to provide an initial burst of sweetness and/or a prolonged sensation of sweetness. Without being limited thereto, such physical forms include free forms, such as spray dried, powdered, beaded forms, encapsulated forms, and mixtures thereof.

In general, an effective amount of intense sweetener may be utilized to provide the level of sweetness desired, and this amount may vary with the sweetener selected. In some embodiments the amount of sweetener maybe present in amounts from 0.001 - 5 wt% of any zone to which it is added, in some examples from 0.001 - 3 wt%, depending upon the sweetener or combination of sweeteners used. Alternatively, the intense sweetener may be present in from 0.001 - 5 wt% of the total sweetener, in some examples from 0.001 - 3 wt%. The exact range of amounts for each type of intense sweetener may be selected by those skilled in the art.

In some embodiments, one or more zones of the comestible of the invention will comprise no, or only low levels of, the so-called "artificial" sweeteners (sweeteners such as saccharin, aspartame, sucralose, neotame and acesulfame potassium). These may be present in the range 0 - 0.005 wt %, alternatively less than 0.005 wt%. In some examples one or more zones of the comestible product, or the entire comestible product will be substantially free of artificial sweetener. This may be desirable, for instance, when a "natural" product is to be prepared. Alternatively, one or more zones of the comestible of the invention will comprise intense sweetener in the range 0 - 0.005 wt%, alternatively less than 0.005 wt% of intense sweetener. In some examples one or more zones of the comestible product, or the entire comestible product will be substantially free of intense sweetener.

### Cooling Agent

Although generally not preferred, a cooling agent may in some embodiments be included in the comestible. Where present it will preferably be present at a level in the range 0.01 - 10 wt% of any zone to which it is added. However, in most embodiments the cooling agent, if present at all, will be present in the range 0 - 0.05 wt% of the comestible. However, it is preferred that the comestible product is substantially free of a cooling agent.

If included, a variety of well-known cooling agents may be employed. For example, among the useful cooling agents are included menthol, xylitol, erythritol, dextrose, sorbitol, menthane, menthone, ketals, menthone ketals, menthone glycerol ketals, substituted p-menthanes, acyclic carboxamides, mono menthyl glutarate, substituted cyclohexanamides, substituted cyclohexane carboxamides, substituted ureas and sulfonamides, substituted menthanols, hydroxymethyl and hydroxymethyl derivatives of p-menthane, 2-mercapto-cyclo-decanone, 2-isopropanyl-5-methylcyclohexanol, hydroxycarboxylic acids with 2-6 carbon atoms, cyclohexanamides, menthyl acetate, menthyl lactate, methyl salicylate, N,2,3-trimethyl-2-isopropyl butanamide (WS-23), N-ethyl-p-menthane-3-carboxamide (WS-3), menthyl succinate, isopulegol, 3,1-menthoxypropane 1,2-diol, glutarate esters, 3-(1-menthoxy)-2-methylpropane-1,2-diol, p-menthane-2,3-diol, p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, 3-(1-menthoxy)ethan-1-ol, 3-(1-menthoxy)propan-1-ol, 3-(1-menthoxy)butan-1-ol, 1-menthylacetic acid N-ethylamide, 1-menthyl-4-hydroxypentanoate, 1-menthyl-3-hydroxybutyrate, N,2,3-trimethyl-2-(1-methylethyl)-butanamide, n-ethyl-t-2-c-6 nonadienamide, N,N-dimethyl menthyl succinamide, substituted p-menthanes, substituted p-menthane-carboxamides, 2-isopropanyl-5-methylcyclohexanol (from Hisamitsu Pharmaceuticals, hereinafter "isopregol"); menthone glycerol ketals (FEMA 3807, trade name FRESCOLAT® type MGA); 3-1-menthoxypropane-1,2-diol (from Takasago, FEMA 3784); and menthyl lactate; (from Haarman & Reimer, FEMA 3748, trade name FRESCOLAT® type ML), WS-30, ·WS-14, Eucalyptus extract (p-Mehtha-3,8-Diol), Menthol (its natural or synthetic derivatives), Menthol PG carbonate, Menthol EG carbonate, Menthol glyceryl ether, N-tertbutyl-p-menthane-3-carboxamide, P-menthane-3-carboxylic acid glycerol ester, Methyl-2-isopryl-bicyclo (2.2.1), Heptane-2-carboxamide; and Menthol methyl ether, and menthyl pyrrolidone carboxylate among others, and combinations thereof. These and other suitable cooling agents are further described in the following U.S. patents: U.S. 4,230,688 and 4,032,661 to Rowsell et al.; 4,459,425 to Amano et al.; 4,136,163 to Watson et al.; 5,266,592 to Grub et al.; and U.S. Patent No. 6,627,233 to Wolf et al. The cooling agents most commonly used in the invention are selected from menthol, xylitol, erythritol, dextrose, sorbitol, menthane, menthone, or combinations thereof.

### Carboxylic Acid Salts

The edible organic salts of monosaccaharide and disaccharide acids may also be present in the inventive comestible. Suitable salts include, by way of example, salts of alkali metal and alkaline earth metal cations, such as calcium, sodium, magnesium, zinc, and potassium salts. The preferred organic acid salts include salts of monocarboxylic organic acids. Salts of monocarboxylic acids having a carbon chain length from C1- C10 are preferred. Salts of monocarboxylic acids having a linear, optionally branched, backbone having a carbon chain length from C1- C10, and ideally from C1- C6, are most preferred.

Particularly preferred salts include at least one of a gluconate and/or a lactate. Suitable salts include, by way of example, calcium lactate, magnesium lactate, sodium lactate, calcium gluconate, magnesium gluconate, and sodium gluconate. Magnesium gluconate is used in a preferred embodiment of the invention.

Specific examples of suitable organic salts for use in the invention include, by way of example, Gluconal KG, Puramex MG, Purasal-P, Purasal Hi-Pure P, and Purasal (all available commercially from Purac of Gorinchem, The Netherlands).

The salts of monocarboxylic acid and dicarboxylic acid, where present, may be present in the range from 0.001 - 1.0 wt% of any zone to which they are added. More preferably, these salts are present in the range from 0.005 - 0.1 wt%. Most preferably, these salts are present in the range from 0.01 - 0.05 wt%.

The salts of monocarboxylic acids and/or dicarboxylic acids may be used alone or in any suitable combination.

### Colourings

Colouring agents may be used in amounts effective to produce the desired colour. The colouring agents may include pigments which may be incorporated in amounts in the range 0-6 wt% of any zone of the comestible to which they are added. For example, titanium dioxide may be incorporated in amounts in the range 0.001 - 2 wt%, and preferably in the range 0.001 - 1 wt% of the zone. The colourants may also include natural food colours and dyes suitable for food, drug and cosmetic applications. A full recitation of all F.D. & C. colourants and their corresponding chemical structures can be found in the Kirk-Othmer Encyclopaedia of Chemical Technology, 3rd Edition, in volume 5 at pages 857-884.

As classified by the United States Food, Drug, and Cosmetic Act (21 C.F.R. 73), colours can include exempt from certification colours (sometimes referred to as natural even though they can be synthetically manufactured) and certified colours (sometimes referred to as artificial), or a combination comprising at least one of the foregoing. In some embodiments, exemplary exempt from certification or natural colours can include, annatto extract, (E160b), bixin, norbixin, astaxanthin, dehydrated beets (beet powder), beetroot red/betanin (E162), ultramarine blue, canthaxanthin (E161g), cryptoxanthin (E161c), rubixanthin (E161d), violanxanthin (E161e), rhodoxanthin (E161f), caramel (E150(a-d)), β-apo-8'-carotenal (E160e), β-carotene (E160a), alpha carotene, gamma carotene, ethyl ester of beta-apo-8 carotenal (E160f), flavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape colour extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), or a combination comprising at least one of the foregoing.

In some embodiments, exemplary certified colours can include FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminium (E173), silver (E174), gold (E175), pigment rubine/lithol rubine BK (E180), calcium carbonate (E170), carbon black (E153), black PN/brilliant black BN (E151), green S/acid brilliant green BS (E142), or a combination comprising at least one of the foregoing. In some embodiments, certified colours can include FD&C aluminium lakes. These consist of the aluminium salts of FD&C dyes extended on an insoluble substrate of alumina hydrate. Additionally, in some embodiments, certified colours can be included as calcium salts.

### Warming Agents

Warming agents may occasionally be present in one or more zones of the inventive comestibles. These may be selected from a wide variety of compounds known to provide the sensory signal of warming to the individual user. These compounds offer the perceived sensation of warmth, particularly in the oral cavity, and often enhance the perception of flavours, sweeteners and other organoleptic components. Useful warming agents include those having at least one allyl vinyl component, which may bind to oral receptors. Examples of suitable warming agents include, but are not limited to: vanillyl alcohol n-butylether (TK-1000, supplied by Takasago Perfumery Company Ltd., Tokyo, Japan); vanillyl alcohol n-propylether; vanillyl alcohol isopropylether; vanillyl alcohol isobutylether; vanillyl alcohol n-aminoether; vanillyl alcohol isoamylether; vanillyl alcohol n-hexylether; vanillyl alcohol methylether; vanillyl alcohol ethylether; gingerol; shogaol; paradol; zingerone; capsaicin; dihydrocapsaicin; nordihydrocapsaicin; homocapsaicin; homodihydrocapsaicin; ethanol; isopropyl alcohol; iso-amylalcohol; benzyl alcohol; glycerine; chloroform; eugenol; cinnamon oil; cinnamic aldehyde; phosphate derivatives thereof; and combinations thereof.

### Tingling Agents

Tingling agents may occasionally be present in one or more zones of the inventive comestibles. These provide a tingling, stinging or numbing sensation to the user. Tingling agents include, but are not limited to: Jambu Oleoresin or para cress (Spilanthes sp.), in which the active ingredient is Spilanthol; Japanese pepper extract (Zanthoxylum peperitum), including the ingredients known as Saanshool-I, Saanshool-II and Sanshoamide; black pepper extract (piper nigrum), including the active ingredients chavicine and piperine; Echinacea extract; Northern Prickly Ash extract; and red pepper oleoresin. In some embodiments, alkylamides extracted from materials such as jambu or sanshool may be included. Additionally, in some embodiments, a sensation is created due to effervescence. Such effervescence is created by combining an alkaline material with an acidic material, either or both of which may be encapsulated. In some embodiments, an alkaline material may include alkali metal carbonates, alkali metal bicarbonates, alkaline earth metal carbonates, alkaline earth metal bicarbonates and mixtures thereof. In some embodiments, an acidic material may include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. Examples of tingling" type sensates can be found in U.S. Patent No. 6,780,443. Tingling agents are described in U.S. Patent No. 6,780,443 to Nakatsu et al., U.S. Patent No. 5,407,665 to McLaughlin et al., U.S. Patent No. 6,159,509 to Johnson et al. and U.S. Patent No. 5,545,424 to Nakatsu et al.

### Taste Potentiators

Any of a variety of substances that function as taste potentiators may be employed in the comestibles described herein, in a single zone, or multiple zones. For instance, suitable taste potentiators include water-soluble taste potentiators, such as, but not limited to, neohesperidin dihydrochalcone, chlorogenic acid, alapyridaine, cynarin, miraculin, glupyridaine, pyridinium-betain compounds, glutamates, such as monosodium glutamate and monopotassium glutamate, neotame, thaumatin, tagatose, trehalose, salts, such as sodium chloride, monoammonium glycyrrhizinate, vanilla extract (in ethyl alcohol), water-soluble sugar acids, potassium chloride, sodium acid sulfate, water-soluble hydrolyzed vegetable proteins, water-soluble hydrolyzed animal proteins, water-soluble yeast extracts, adenosine monophosphate (AMP), glutathione, water-soluble nucleotides, such as inosine monophosphate, disodium inosinate, xanthosine monophosphate, guanylate monophosphate, alapyridaine (N-(1-carboxyethyl)-6-(hydroxymethyl)pyridinium-3-ol inner salt, sugar beet extract (alcoholic extract), sugarcane leaf essence (alcoholic extract), curculin, strogin, mabinlin, gymnemic acid, 2-hydroxybenzoic acid (2-HB), 3-hydroxybenzoic acid (3-HB), 4-hydroxybenzoic acid (4-HB), 2,3-dihydroxybenzoic acid (2,3-DHB), 2,4-dihydroxybenzoic acid (2,4-DHB), 2,5-dihydroxybenzoic acid (2,5-DHB), 2,6-dihydroxybenzoic acid (2,6-DHB), 3,4-dihydroxybenzoic acid (3,4-DHB), 3,5-dihydroxybenzoic acid (3,5-DHB), 2,3,4-trihydroxybenzoic acid (2,3,4-THB), 2,4,6-trihydroxybenzoic acid (2,4,6-THB), 3,4,5-trihydroxybenzoic acid (3,4,5-THB), 4-hydroxyphenylacetic acid, 2-hydroxyisocaproic acid, 3-hydroxycinnamic acid, 3-aminobenzoic acid, 4-aminobenzoic acid, 4-methoxysalicylic acid and combinations thereof.

The potentiator acts on an active substance to enhance its perception in some manner. In some embodiments, a mixture of at least one active substance and at least one taste potentiator is encapsulated, rather than encapsulating the taste potentiator or the active substance alone. The encapsulant may be selected to delay or increase the rate of release of the mixture of components.

In embodiments including an encapsulated mixture of active(s) and potentiator(s), the active substance(s) may be present in amounts of 1 - 95 wt% of any zone of the comestible to which they are added, more specifically 5 - 30 wt%. The taste potentiator(s) (whether encapsulated or not) may be present in amounts of 0.01 - 12 wt% of the zone, more specifically 0.1-5 wt%. The encapsulant may be present in amounts of 1- 95 wt%, more specifically 10-60 wt% of the relevant zone or zones.

Some embodiments may include a mixture of at least one encapsulated taste potentiator and at least one taste potentiator in its free form. The encapsulated and unencapsulated taste potentiators may be the same or different. The mixture of encapsulated and unencapsulated taste potentiators may be combined with one or more active substances to provide a potentiator composition.

Some other embodiments provide compositions that modulate the activity of taste receptor cells in a mammal. Such compositions may include at least one active substance and at least one taste potentiator, as described above. These components may be encapsulated or unencapsulated, also as described above. The taste potentiator(s) may modulate the activity of taste receptor cells upon consumption of the composition. More specifically, taste is perceived through sensory cells located in the taste buds. Different signaling mechanisms sense the primary tastes of salty, sour, sweet, bitter and umami. Eventually a nerve impulse is triggered in the brain that is sensed as one of these primary tastes.

Taste potentiators function by modulating the activity of taste receptor cells at some point in this taste signaling pathway. For instance, in some cases, taste potentiators may bind to taste receptors, such as, for example, sweet taste receptors, which thereby enhances the perception of the sweet taste. In other embodiments, for example, taste potentiators may block taste receptors, such as, for example bitter receptors, which suppress the perception of a bitter taste and thereby enhances the perception of a sweet taste. Taste potentiator(s), therefore, modulate the activity of taste receptor cells in mammals, which thereby enhances the perception of a given taste. This activity may enhance the perception of an active substance contained in the composition when consumed in conjunction with a taste potentiator.

### Umami

Compounds that provide umami or savoury flavour may include monosodium glutamate (MSG), glutamic acid, glutamates, aspartate, free amino acids, IMP (disodium 5'-inosine monophosphate) and GMP (disodium 5'-guanosine monophosphate), compounds that stimulate T1R1 and T1R3 receptors, mushroom flavour, fermented fish flavour, and muscle flavours, such as beef, chicken, pork, ostrich, venison and buffalo. The level of umami present in any comestible, or zone of comestible may be modified without undue experimental burden on the person skilled in the art. However, it is preferably present in from 0.001 - 3 wt% of the zone, often 0.001 - 1 wt%.

### Kokumi

Substances that impart kokumi may include a mixture selected from: (1) gelatin and tropomyosin and/or tropomyosin peptides; (2) gelatin and paramyosin; and (3) troponin and tropomyosin and/or tropomyosin peptides, as disclosed in U.S. Patent No. 5,679,397 to Kuroda et al., referred to above. The level of kokumi present in any comestible, or zone of comestible may be modified without undue experimental burden on the person skilled in the art. However, it is preferably present in from 0.001 - 3 wt% of the zone, often 0.001 - 1 wt%.

### Saltiness

Compounds that provide saltiness may include conventional salts, such as sodium chloride, calcium chloride, potassium chloride, 1-lysine and combinations thereof. The level of salt compound present in any comestible, or zone of comestible may be modified without undue experimental burden on the person skilled in the art. However, it is preferably present in from 0.001 - 3 wt% of the zone, often 0.001 - 1 wt%.

### Lubricants

Lubricants also may be added in some embodiments to improve the smoothness of the comestible, such as, for example hard candy embodiments. Smoothness also is a characteristic that leads to an increased perception of mouth-moistening upon consumption. Suitable lubricants include, but are not limited to, fats, oils, aloe vera, pectin and combinations thereof. Where present the lubricant may be included at a level in the range 0.5 - 15 wt%, optionally 1-10 wt% of any zone to which it is added.

### Breath Fresheners

Breath-freshening agents, in addition to the flavours and cooling agents described hereinabove, may include a variety of compositions with odour-controlling properties. Such breath-freshening agents may include, without limitation, cyclodextrin and magnolia bark extract. The breath-freshening agents may further be encapsulated to provide a prolonged breath-freshening effect. Examples of malodour-controlling compositions are included in U.S. Patent No. 5,300,305 to Stapler et al. and in U.S. Patent Application Publication Nos. 2003/0215417 and 2004/0081713. In some embodiments, the breath freshener will comprise from 0.01 - 20 wt%, more preferably 0.02 - 8 wt% of any zone to which they are added.

### Oral Care Products

A variety of oral care products also may be included in one or more zones of some embodiments of the instant comestible products. Such oral care products may include tooth whiteners, stain removers, anti-calculus agents, remineralisation agents and antiplaque agents. Oral care agents that may be used include those actives known to the skilled artisan, such as, but not limited to, surfactants, breath-freshening agents, anti-microbial agents, antibacterial agents, oral malodour control agents, fluoride compounds, quaternary ammonium compounds, remineralisation agents and combinations thereof. Examples of these include, but are not limited to hydrolytic agents including proteolytic enzymes, abrasives such as hydrated silica, calcium carbonate, sodium bicarbonate and alumina, other active stain-removing components such as surface-active agents, such as anionic surfactants such as sodium stearate, sodium palminate, sulfated butyl oleate, sodium oleate, salts of fumaric acid, glycerol, hydroxylated lecithin, sodium lauryl sulfate and chelators such as polyphosphates, which are typically employed in dentifrice compositions as tartar control ingredients. Also included are tetrasodium pyrophosphate and sodium tri-polyphosphate, sodium tripolyphosphate, xylitol, hexametaphosphate, and an abrasive silica. Further examples are included in the following U.S. Patents: U.S. Patent Nos. 5,227,154 to Reynolds, 5,378,131 to Greenberg and 6,685,916 to Holme et al. Suitable oral care actives such as remineralisation agents, antimicrobials, and tooth-whitening agents are described in assignee's co-pending U.S. Patent Application No. 10/901,511, filed July 29, 2004 and entitled "Tooth-Whitening Compositions and Delivery Systems Therefore," and mixtures thereof.

The oral care product will typically be present in the range 0.01 - 40 wt% of any zone in which is it present, often 0.01 - 20 wt%, in some examples 0.03 - 15 wt%, in preferred examples 0.05 -10 wt% of the zone or zones.

Suitable surfactants include, but are not limited to, salts of fatty acids selected from the group consisting of C₈-C₂₄, palmitoleic acid, oleic acid, eleosteric acid, butyric acid, caproic acid, caprylic acid, capric acid, lauric acid, myristic acid, palmitic acid, stearic acid, ricinoleic acid, arachidic acid, behenic acid, lignoceric acid, cerotic acid, sulfated butyl oleate, medium and long chain fatty acid esters, sodium oleate, salts of fumaric acid, potassium glomate, organic acid esters of mono- and diglycerides, stearyl monoglyceridyl citrate, succistearin, dioctyl sodium sulfosuccinate, glycerol tristearate, lecithin, hydroxylated lecithin, sodium lauryl sulfate, acetylated monoglycerides, succinylated monoglycerides, monoglyceride citrate, ethoxylated mono- and diglycerides, sorbitan monostearate, calcium stearyl-2-lactylate, sodium stearyl lactylate, lactylated fatty acid esters of glycerol and propylene glycerol, glycerol-lactoesters of C₈-C₂₄ fatty acids, polyglycerol esters of C₈-C₂₄ fatty acids, propylene glycol alginate, sucrose C₈-C₂₄ fatty acid esters, diacetyl tartaric and citric acid esters of mono- and diglycerides, triacetin, sarcosinate surfactants, isethionate surfactants, tautate surfactants, pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and combinations thereof.

Suitable anti-calculus agents include, but are not limited to, pyrophosphates, triphosphates, polyphosphates, polyphosphonates, dialkali metal pyrophosphate salt, tetra alkali polyphosphate salt, tetrasodium pyrophosphate, tetrapotassium pyrophosphate, sodium tripolyphosphate and combinations thereof.

Suitable remineralisation agents include, but are not limited to casein phosphopeptide-amorphous calcium phosphate, casein phosphoprotein-calcium phosphate complex, casein phosphopeptide-stabilized calcium phosphate, and combinations thereof.

### Pharmaceuticals

A variety of drugs, including medications, herbs, and nutritional supplements may also be included in one or more zones of the inventive comestible product. Examples of useful drugs include ace-inhibitors, antianginal drugs, anti-arrhythmias, antiasthmatics, anti-cholesterolemics, analgesics, anesthetics, anti-convulsants, anti-depressants, anti-diabetic agents, anti-diarrhoea preparations, antidotes, anti-histamines, anti-hypertensive drugs, anti-inflammatory agents, anti-lipid agents, antimanics, anti-nauseants, anti-stroke agents, anti-thyroid preparations, anti-tumour drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra®, fertility agents, gastrointestinal agents, homeopathic remedies, hormones, hypercalcemia and hypocalcaemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocryptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumour drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, antiasthmatics, cough suppressants, mucolytics, DNA and genetic modifying drugs, and combinations thereof.

Examples of active ingredients contemplated for use in the inventive comestibles include antacids, H2-antagonists, and analgesics. For example, antacid dosages can be prepared using the ingredients calcium carbonate alone or in combination with magnesium hydroxide, and/or aluminium hydroxide. Moreover, antacids can be used in combination with H2-antagonists.

Analgesics include opiates and opiate derivatives, such as Oxycontin, ibuprofen, aspirin, acetaminophen, and combinations thereof that may optionally include caffeine.

Other drug ingredients for use in embodiments include anti-diarrhoeal such as immodium AD, anti-histamines, anti-tussives, decongestants, vitamins, and breath-fresheners. Also contemplated for use herein are anxiolytics such as Xanax; anti-psychotics such as clozaril and Haldol; non-steroidal anti-inflammatories (NSAID's) such as ibuprofen, naproxen sodium, Voltaren and Lodine, anti-histamines such as Claritin, Hismanal, Relafen, and Tavist; anti-emetics such as Kytril and Cesamet; bronchodilators such as Bentolin, Proventil; anti-depressants such as Prozac, Zoloft, and Paxil; anti-migraines such as Imigra, ACE-inhibitors such as Vasotec, Capoten and Zestril; anti-Alzheimer's agents, such as Nicergoline; and CaH-antagonists such as Procardia, Adalat, and Calan.

Moreover, some embodiments of the invention can include H2-antagonists. Examples of suitable H2-antagonist include cimetidine, ranitidine hydrochloride, famotidine, nizatidien, ebrotidine, mifentidine, roxatidine, pisatidine and aceroxatidine.

Active antacid ingredients include, but are not limited to, the following: aluminium hydroxide, dihydroxyaluminium aminoacetate, aminoacetic acid, aluminium phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminium mono-ordibasic calcium phosphate, tricalcium phosphate, potassium bicarbonate, sodium tartrate, sodium bicarbonate, magnesium aluminosilicates, tartaric acids and salts.

Preferably, the pharmaceuticals are present at levels of approximately 50 micrograms to 500 milligrams. The specific levels will depend on the active ingredient. For example, aspirin would be present at a level of 325 milligrams per 2.8/gram serving.

### Nutritional Supplements

A variety of other nutritional supplements also may be included in one or more zones of the comestible product. Virtually any vitamin or mineral may be included. For example, vitamin A, vitamin C, vitamin D, vitamin E, vitamin K, vitamin B₆, vitamin B₁₂, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used. Examples of nutritional supplements are set forth in U.S. Patent Application Publication Nos. 2003/0157213 A1, 2003/0206993 and 2003/0099741 A1.

Various herbs also may be included such as those with various medicinal or dietary supplement properties. Herbs are generally aromatic plants or plant parts that can be used medicinally or for flavouring. Suitable herbs can be used singly or in various mixtures. Commonly used herbs include Echinacea, Goldenseal, Calendula, Aloe, Blood Root, Grapefruit Seed Extract, Black Cohosh, Cranberry, Ginko Biloba, St. John's Wort, Evening Primrose Oil, Yohimbe Bark, Green Tea, Maca, Bilberry, Lutein, and combinations thereof.

The amount of vitamin, mineral or herb present in the comestible will depend upon the nature of the supplement and its potency. The determination of the appropriate level would possible by the skilled person without undue experimental burden. However, the nutritional supplements will typically be present in the range, 0-5 wt%, often in the range 0.001-3 wt% of any zone to which they are added.

### Antibacterial Agents

Suitable antibacterial agents include, but are not limited to, chlorhexidine, alexidine, quaternary ammonium salts, benzethonium chloride, cetyl pyridinium chloride, 2,4,4'-trichloro-2'-hydroxy-diphenyl ether (triclosan) and combinations thereof.

Suitable anti-microbial agents include, but are not limited to, cetylpyridinium chloride, zinc compounds, copper compounds and combinations thereof. Where present, the antimicrobial will typically be present in the range 0.001 - 5 wt% of any zone to which it is added.

### Preservatives

Preservatives, including antimicrobials, can be added to one or more zones of the composition to provide freshness and to prevent the unwanted growth of bacteria, molds, fungi, or yeast. Preferably, any preservative will be added to at least the zones which are partly in contact with the outermost surface of a multizone product. The addition of a preservative, including antioxidants, may also be used to maintain the composition's colour, flavour, or texture. Any suitable preservatives for use in food products can be incorporated into the compositions. Examples of suitable preservatives include benzoic acid alkali metal salts (e.g., sodium benzoate), sorbic acid alkali metal salts (e.g., potassium sorbate), ascorbic acid (Vitamin C), citric acid, calcium propionate, sodium erythorbate, sodium nitrite, calcium sorbate, butylated hydroxyanisole (BHA), butylated hydroxytoluene (BHT), ethylenediaminetetraacetic acid (EDTA), tocopherols (Vitamin E), straight chain polyphosphates, or a combination comprising at least one of the foregoing preservatives.

The composition may contain the preservative or preservative combination in an amount of 0.0001 - 0.10 wt%, specifically 0.001 - 0.08 wt%, more specifically 0.005 - 0.05 wt%, and yet more specifically 0.01 - 0.04 wt% of any zone to which it is added.

### Antioxidant

Antioxidants may include materials that scavenge free radicals. In some embodiments, antioxidants can include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavinoids, and combinations thereof.

The composition may contain the antioxidant in an amount of 0.0001 - 0.10 wt%, specifically 0.001 - 0.08 wt%, more specifically 0.005 -0.05 wt%, and yet more specifically 0.01 - 0.04 wt% of any zone to which it is added.

### Use in Candy

As discussed above, the comestible of the invention may be a candy, often a chewy candy although a wide variety of candies are intended to fall within the scope of the invention. Candies are sweet confectioneries which are made from sugar and solvent solutions, in most cases from sugar and water. A range of optional ingredients may be added to these, as outlined above. It is particularly common for flavourings and colourings to be present in all types of candy.

### Use in Chewing Gum

As discussed previously, the invention has application in chewing gum technology. As used herein, the term "chewing gum" is intended to relate to any gum composition, including "bubble gums". Chewing gum typically comprises a water insoluble gum base and a water soluble phase comprising primarily sweeteners and flavours.

The elastomers (rubbers) employed in the gum base of the chewing gum will vary greatly depending upon various factors such as the type of gum base desired, the consistency of gum composition desired and the other components used in the composition to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base compositions include, without limitation, natural substances (of vegetable origin) such as chicle, natural rubber, crown gum, nispero, rosidinha, jelutong, perillo, niger gutta, tunu, balata, guttapercha, lechi capsi, sorva, gutta kay, and the like, and combinations thereof. Examples of synthetic elastomers include, without limitation, styrene-butadiene copolymers (SBR), polyisobutylene, isobutylene-isoprene copolymers, polyethylene, polyvinyl acetate and the like, and combinations thereof. Specific examples of elastomers include polyisobutylene, styrene butadiene rubber, butyl rubber, and combinations thereof.

Additional useful polymers include: polybutylmethacrylate/acrylic acid copolymers, polyvinylacetate/vinylalcohol copolymers, microcrystalline cellulose, sodium carboxymethyl cellulose, hydroxylpropylmethyl cellulose, crosslinked cellulose acetate phthalate, crosslinked hydroxyl methyl cellulose polymers, zein, crosslinked polyvinyl pyrrolidone, polymethylmethacrylate/acrylic acid copolymers, copolymers of lactic acid, polyhydroxyalkanoates, plasticized ethylcellulose, polyvinyl acetatephthalate and combinations thereof.

In general, the elastomer employed in the gum base may have an average molecular weight of at least about 200,000. Desirably, the elastomer employed in the gum base has an average molecular weight from about 200,000 to about 2,000,000.

In some embodiments, it is particularly useful to include an elastomer composition including a predominant amount of a material selected from polyisobutylene, butyl rubber, butadiene-styrene rubber and combinations thereof, the elastomer composition having an average molecular weight of at least about 200,000; and a mastication processing aid, wherein the addition of the non-stick and/or degradability inducing component maintains the glass transition temperature of the elastomer within a three degree (3°) range, i.e., +/- three degrees. By "predominant" is meant that the composition includes greater than 50 - 98 wt% of a material selected from polyisobutylene, butyl rubber, butadiene-styrene rubber and combinations thereof.

The amount of elastomer employed in the gum base may vary depending upon various factors such as the type of gum base used, the consistency of the gum composition desired and the other components used in the composition to make the final chewing gum product. In general, the elastomer may be present in the gum base in an amount from 1 - 30 wt% of the gum base. Desirably, the elastomer is present in an amount from 2 - 15 wt% of the gum base. More desirably, the elastomer is present in the gum base in an amount from 3 - 10 wt% of the gum base.

In some embodiments, the elastomer will be present in the gum base in an amount from 10 - 60 wt%, desirably from 35 - 40 wt%.

In some embodiments, the chewing gum base may include a texture-modifier. In general, the texture-modifier has a molecular weight of at least about 2,000. The texture-modifier may include a vinyl polymer, for example, polyvinyl acetate, polyvinyl laurate acetate, polyvinyl alcohol or mixtures thereof. Desirably, the texture-modifier is present in an amount from 15 - 70 wt% of the gum base. More desirably, the texture-modifier is present in an amount from 20 - 60 wt% of the gum base. Most desirably, the texture-modifier is present in an amount from 30 - 45 wt% of the gum base.

In addition to the components set out above, the gum base may include a variety of other ingredients, such as components selected from elastomer solvents, emulsifiers, plasticizers, fillers, and mixtures thereof. As mentioned above, the use of elastomer solvents is not needed to masticate the rubber during the manufacturing process. It may be present in limited amounts, but can lessen from the non-stick properties of the invention if used in amounts above 5 wt% of the gum base. In certain embodiments of the invention, elastomer solvents may be used in amounts of 4 - 5 wt% of the gum base to provide non-stick properties which are sufficient to provide non-stick properties to teeth, dentures, oral implants and other oral prosthetics.

In some embodiments, the gum base may also contain less than conventional amounts of elastomer solvents to aid in softening the elastomer component. In particular, in some embodiments, such solvents are not required, but may be used in limited amounts along with the non-stick and/or degradability inducing components. By less than conventional amounts is meant that the elastomer solvent is employed in the gum base, for example, in amounts from 0 - 5.0 wt% and preferably from 0.1 - 3.0 wt% of the gum base. In some embodiments, the gum base includes a maximum of about 5.0 wt% of an elastomer solvent. In other embodiments, the gum base is free of added elastomer solvents. In some embodiments the gum base is also free of added waxes.

In other embodiments, conventional amounts of elastomer solvents are incorporated in the gum bases to aid in softening the elastomer component.

Such elastomer solvents may include those elastomer solvents known in the art, for example, terpinene resins such as polymers of alpha-pinene or beta-pinene, methyl, glycerol and pentaerythritol esters of rosins and modified rosins and gums such as hydrogenated, dimerized and polymerized rosins, and mixtures thereof. Examples of elastomer solvents suitable for use herein may include the pentaerythritol ester of partially hydrogenated wood and gum rosin, the pentaerythritol ester of wood and gum rosin, the glycerol ester of wood rosin, the glycerol ester of partially dimerized wood and gum rosin, the glycerol ester of polymerized wood and gum rosin, the glycerol ester of tall oil rosin, the glycerol ester of wood and gum rosin and the partially hydrogenated wood and gum rosin and the partially hydrogenated methyl ester of wood and rosin, and the like, and mixtures thereof.

Desirably, the incorporation of an elastomer solvent in the gum base does not interfere with the non-stick inducing components of the gum base and/or with the ability of the gum base to degrade. In particular, in some embodiments where non-stickiness or reduced stickiness is desired, the elastomer solvent desirably softens the gum base without contributing to stickiness. Moreover, the Tg of the gum base desirably does not change more than +/- three (3°) upon incorporation of the elastomer solvent in the gum base in some embodiments where non-stickiness or reduced stickiness is desired.

In some embodiments, when a hydrophilic precursor component is incorporated into the inventive gum bases, an elastomer solvent may or may not be present. In particular, in some embodiments when a hydrophilic precursor component is used, the elastomer solvent is present is less than conventional amount, i.e., in amounts from 0 - 5 wt% and preferably from 0.1 - 3 wt% of the gum base. In other embodiments when a hydrophilic precursor component is used, the elastomer solvent is present in conventional amounts, i.e., in amounts greater than 5 wt% for the gum base. For example, the elastomer solvent may be present in an amount from 2.0 - 15 wt% and, more particularly, from 5 - 15 wt% of the gum base and, even more particularly, in amounts from 7 - 11 wt% of the gum base.

In some embodiments, the elastomer solvent employed may have at least one hydrophilic portion and at least one hydrophobic portion such that the hydrophilic portion orients inwardly within a gum base and such that the hydrophilic portion orients outwardly within a gum base made from elastomers. Suitable elastomer solvents having at least one hydrophilic portion and at least on hydrophobic portion include, for example, methyl ester liquid rosin. In some embodiments, it is especially useful to incorporate a methyl ester liquid rosin in relatively low amounts. Methyl ester liquid rosin interferes less with the non-stick and/or degradability inducing components as compared to other resins, but yet acts to increase softening of the gum base without contributing to increased stickiness when used in combination with the non-stick inducing component.

Desirably, in some embodiments, a methyl ester liquid rosin is incorporated in a gum base in an amount from 0.5 - 5.0 wt% of the gum base. More desirably, a methyl ester liquid rosin is incorporated in a gum base in an amount from 1.0 - 3.0 wt% of the gum base.

The gum base also may include emulsifiers which aid in dispersing the immiscible components of the gum base into a single stable system. The emulsifiers useful in this invention include glyceryl monostearate, lecithin, fatty acid monoglycerides, diglycerides, propylene glycol monostearate, and the like, and mixtures thereof. In some embodiments, the emulsifier may be employed in amounts from 0 - 50 wt% and, more specifically, from 2 - 7 wt% of the gum base. In other embodiments, the emulsifier may be employed in amounts from 2 - 15 wt% and, more specifically, from 7 - 11 wt% of the gum base.

The gum base also may include plasticizers or softeners to provide a variety of desirable textures and consistency properties. Because of the low molecular weight of these ingredients, the plasticizers and softeners are able to penetrate the fundamental structure of the gum base making it plastic and less viscous. Useful plasticizers and softeners include triacetin (glyceryl triacetate), lanolin, palmitic acid, oleic acid, stearic acid, sodium stearate, potassium stearate, glyceryl triacetate, glyceryl lecithin, glyceryl monostearate, propylene glycol monostearate, acetylated monoglyceride, glycerine, waxes, and the like, and mixtures thereof. Other softeners include carob, tragacanth, locust bean, and carboxymethyl cellulose. In some embodiments, the aforementioned plasticizers and softeners are generally employed in the gum base in amounts up to about 20 wt% of the gum base, and more specifically in amounts from 2 - 12 wt% of the gum base. In other embodiments, the plasticizers and softeners are generally employed in the gum base in amounts up to about 20 wt% of the gum base and, more specifically, in amounts from 9 - 17 wt% of the gum base.

Plasticizers also include hydrogenated vegetable oils, such as soybean oil and cottonseed oils, which may be employed alone or in combination. These plasticizers provide the gum base with good texture and soft chew characteristics. These plasticizers and softeners are generally employed in amount from 5 - 14 wt% and, more specifically, in amounts from 5 - 13.5 wt% of the gum base.

Suitable waxes, include for example, natural and synthetic waxes; hydrogenated vegetable oils, petroleum waxes such as polyurethane waxes, polyethylene waxes, paraffin waxes, microcrystalline waxes, fatty waxes, sorbitan monostearate, tallow, propylene glycol, mixtures thereof, and the like. Wax can be present in the gum base in an amount from 1-15 wt% of the gum base. In some embodiment, when used, the wax is desirably present in an amount from 2 - 10 wt% of the gum base and, more desirably, is present in an amount from 3 - 8 wt% of the gum base. In other embodiments when wax is used, the wax may be present in the gum base in an amount from 6 - 10% and, more desirably, from 7 - 9.5 wt% of the gum base. In some embodiments, the gum base includes a maximum of about 8 wt% of a wax. In other embodiments, the gum base is free of added wax.

In some embodiments when wax is present, the waxes employed may have a melting point below about 60°C and, more desirably, between about 45°C and about 55°C. The wax having a low melting point may be, for example, a paraffin wax. In addition to low melting point waxes, in some embodiments, waxes having a higher melting point may be used in the gum base in amounts up to about 5 wt% of the gum base. Such high melting waxes include, for example, beeswax, vegetable wax, candelilla wax, carnuba wax, most petroleum waxes, and the like and mixtures thereof.

Anhydrous glycerin also may be employed as a softening agent, such as the commercially available United States Pharmacopeia (USP) grade. Glycerin is a syrupy liquid with a sweet warm taste and has a sweetness of about 60% of that of cane sugar. Because glycerin is hygroscopic, the anhydrous glycerin may be maintained under anhydrous conditions throughout the preparation of the chewing gum composition.

In some embodiments, the gum base of this invention may include bulking agents that are water-insoluble and/or mineral-based. In particular, the gum base of this invention also may include effective amounts of bulking agents such as mineral adjuvants which may serve as fillers and textural agents. Useful mineral adjuvants include calcium carbonate, magnesium carbonate, alumina, aluminium hydroxide, aluminium silicate, talc, starch, tricalcium phosphate, dicalcium phosphate, calcium sulfate, atomite, and the like, and mixtures thereof. These fillers or adjuvants may be used in the gum base compositions in various amounts. The filler may be present in an amount from 0 - 60 wt% of the gum base and/or composition and, more specifically, from 0 - 50 wt% and, even more specifically, from 0 - 40 wt% of the gum base and/or chewing gum composition. In some embodiments, the filler may be present in an amount from 0 - 30 wt% of the gum base and/or chewing gum composition. Moreover, in some embodiments, the amount of filler will be from 0 - 15 wt% of the gum base and/or chewing gum composition and, more specifically, from 3 - 11 wt% of the gum base and/or chewing gum composition. In other embodiments, the amount of filler, when used, may be present in an amount from 15 - 40 wt% and, desirably, from 20 - 30 wt% of the gum base.

In some embodiments, the gum base also may include at least one hydrophilic, water-absorbing polymer to help reduce the stickiness of the gum base and any resultant gum product made from the gum base. Suitable hydrophilic, water-absorbing polymers include the following: native and modified starches; chemically modified cellulose, including methyl cellulose, ethyl cellulose, carboxymethyl cellulose, hydroxypropyl cellulose; gums including xanthan gum, carrageenan gum, guar gum, gum arabic, locust bean gum, curdlan, arabinoxylan, agara, and alginates; and pectin and gelatin.

In general, at least one hydrophilic, water-absorbing polymer is included in an amount from 0.1 - 10 wt% of the gum base. Desirably, at least one hydrophilic, water-absorbing polymer is present in an amount from 2 - 8 wt% of the gum base. More desirably, at least one hydrophilic, water-absorbing polymer is present in an amount from 3 - 6 wt% of the gum base.

In some embodiments, at least one antioxidant may be present in the chewing gum bases. Desirably, the antioxidant is water-soluble. Suitable antioxidants include, for example, butylated hydroxytoluene (BHT), butylated hydroxyanisole (BHA), propyl gallate, vitamin C, vitamin E and mixtures thereof. When an antioxidant is included in the gum base, the antioxidant is generally present in an amount from 0.01 - 0.3 wt% of the gum base. Desirably, the antioxidant is included in the gum base in an amount from 0.05 - 0.1 wt% of the gum base. When incorporated in embodiments together with the agent capable of degrading the elastomer, it is desirable to keep the antioxidant down to lower amounts to prevent any interference with free radicals which may be generated by photosensitizers.

In some embodiments, the chewing gum compositions include at least one elastomer and at least one agent capable of changing the molecular weight of the elastomer over time, such as by degrading the elastomer or increasing the molecular weight of the elastomer.

In some embodiments, a chewing gum base as discussed above may be incorporated in a chewing gum composition in an amount from 5 - 95 wt%. More desirably, a chewing gum base may be present in an amount from 28 - 42 wt% of the total chewing gum composition, and even more specifically, the range may be from 28 - 30 wt% of the total chewing gum composition. In the case of centre-filled chewing gum compositions, this weight percent may be based on the gum region rather than the centre-filled region.

Any of the optional components described above may be incorporated into the chewing gum comestible, as well as other conventional additives known to one having ordinary skill in the art may be incorporated into the chewing gum base of the chewing gum compositions or any coating that the chewing gum product may contain.

### Preparation

The comestible of the invention may be prepared by incorporating galactose into the comestible, for instance, as a glucose substitute, using conventional techniques. Often, the incorporation will be selected from, coating the comestible with galactose, mixing galactose into the comestible, and including one or more galactose containing zones in the comestible. In preferred embodiments the comestible will be a chewing gum or candy which may be centre-filled or hard coated or both.

Where the comestible is a solid, or is sufficiently solid to hold a defined shape, the comestible may have any shape; geometric and irregular shapes are preferred. In some embodiments the comestible may have smooth or rounded edges and/or corners. In such embodiments, the comestible may be, for instance, a square, circular or diamond-shaped, however, in this embodiment the edges are rounded to provide a smooth comestible. Another manner of lending smoothness to the comestibles is to deposit the comestible composition into molds during the manufacturing process.

### Candy

The candy of the invention is made using conventional techniques well known to the person skilled in the art. For the chewy candy of the preferred embodiment of the invention, the candy ingredients are typically combined at elevated temperature with stirring until the mixture is substantially uniform in appearance. The mixture is then cooked until the moisture contents drops to within the range 0 - 10 wt%, more often within the range 1 - 8 wt% of the candy composition. As noted above, the candy comestible may comprise more than one zone, where this is the case, this description describes the production of the candy core. Examples of other zones which may be present in combination with the candy core are described below.

The typical cooking temperatures may vary over the course of the cooking process, for instance, the temperature may be increased once the solid components of the candy have dissolved to encourage the evaporation of the liquid components, thereby reducing the moisture. Often the cooking temperature will be in the range 50 - 175 °C, often in the range 75 - 150°C. In some embodiments the cooking temperature will start at a temperature in the range 75 - 115 °C and be increased after dissolution of the ingredients to a temperature in the range 115 - 170 °C. The use of heat is advantageous as it decreases the viscosity of the mixture, facilitating combination of the ingredients and ease of handling. Where a particularly chewy candy is desired, cooking temperatures should be reduced to allow for the retention of a greater amount of water in the composition.

Once the desired moisture level has been obtained, the candy mixture will be shaped. This may be, for instance, by pouring the candy composition into a mould and allowing the composition to cool before cutting, or preparation by extrusion processes known to the person skilled in the art. Often the candy will be placed in a sizing roll prior to cutting into candy units. The individual units may then be wrapped and/or coated as appropriate.

### Chewing Gum

The chewing gum compositions may be formed into a variety of shapes and sizes and may take various product forms, including without limitation, sticks, slabs, chunks, balls, pillows, tablet, pellet, centre-filled, pressed tablet, deposited, compressed chewing gum or any other suitable format, as well as coated and uncoated forms.

The gum base may be prepared using methods of processing an elastomer for use in a gum base without substantially changing the Tg of the gum base as measured by differential scanning calorimetry (DSC). Such methods include the step of mixing at least one elastomer and at least one fat.

Differential scanning calorimetry (DSC) is a thermoanalytical technique in which the difference in the amount of heat required to increase the temperature of a sample and reference are measured as a function of temperature. The basic principle underlying this technique is that, when the sample undergoes a physical transformation such as phase transitions, more (or less) heat will need to flow to it than the reference to maintain both at the same temperature. Whether more or less heat must flow to the sample depends on whether the process is exothermic or endothermic. For example, as a solid sample melts to a liquid it will require more heat flowing to the sample to increase its temperature at the same rate as the reference. This is due to the absorption of heat by the sample as it uridergoes the endothermic phase transition from solid to liquid. Likewise, as the sample undergoes exothermic processes (such as crystallization) less heat is required to raise the sample temperature. By observing the difference in heat flow between the sample and reference, differential scanning calorimeters are able to measure the amount of energy absorbed or released during such transitions. DSC is used to observe more subtle phase changes, such as glass transitions.

Alternatively, the gum base may be prepared by processing a solid elastomer the steps comprising: providing a solid elastomer composition suitable for use in a chewing gum base and combining with the solid elastomer composition a non-stick and/or degradability inducing component including at least one fat having an HLB range of about 3.5 to about 13. In such methods, the non-stick and/or degradability inducing component is present in amounts sufficient to permit mastication of the solid elastomer composition into a homogenous mass.

Some embodiments extend to methods of making a chewing gum base. In some embodiments, the methods of making a chewing gum base include providing at least one elastomer and mixing at least one non-stick and/or degradability inducing component with the elastomer to form a chewing gum base, wherein the at least one non-stick and/or degradability inducing component softens the elastomer without causing the chewing gum base to become sticky. In such embodiments, the chewing gum base has reduced stickiness in the presence of the non-stick and/or degradability inducing component as compared to in the absence of the non-stick and/or degradability inducing component.

In additional embodiments, the methods of making a chewing gum base include processing an elastomer for use in a gum base without substantially changing the Tg of the gum base as measured by DSC by mixing at least one elastomer and at least one fat or oil.

Moreover, in further embodiments, the methods of making a chewing gum base include providing a solid elastomer composition suitable for use in a chewing gum base and combining with the solid elastomer composition a non-stick and/or degradability inducing component that includes at least one fat or oil having an HLB range of about 3.5 to about 13. In such methods, the non-stick and/or degradability inducing component is present in amounts sufficient to permit processing of the solid elastomer composition into a softened, processable mass.

In some embodiments, the above-described methods of making a chewing gum base may be carried out in the presence of lower than conventional amounts of elastomer solvent. In such embodiments, the elastomer solvent includes a maximum of about 5.0% of the gum base. Desirably, an elastomer solvent can be mixed with an elastomer and non-stick and/or degradability inducing component to soften the elastomer without causing the resultant chewing gum base to become sticky.

In other embodiments, the above-described methods of making a chewing gum base are carried out in the absence of added elastomer solvent.

The manner in which the gum base components are mixed is not critical and such mixing is performed using standard apparatuses known to those skilled in the art. In a typical method, at least one elastomer is admixed with at least one mastication processing aid, which for purposes of the invention includes one or more non-stick and/or degradability inducing components, and agitated for a period of from 1 to 30 minutes. The remaining ingredients, such as the texture-modifier and/or softener are then admixed, either in bulk or incrementally, while the gum base mixture is blended again for 1 to 30 minutes.

The products may be prepared using standard techniques and equipment known to those skilled in the art, which processes generally involve melting the gum base, incorporating the desired ingredients while mixing and forming the batch into individual chewing gum pieces. The apparatus useful in accordance with the embodiments described herein includes mixing and heating apparatuses well-known in the chewing gum manufacturing arts, and therefore the selection of the specific apparatus will be apparent to the artisan. For general chewing gum preparation processes which are useful in some embodiments see U.S. Patent Nos. 4,271,197 to Hopkins et al., 4,352,822 to Cherukuri et al. and 4,497,832 to Cherukuri et al.

### Centre-filling Techniques

Some embodiments extend to methods of making centre-filled compositions. The improved compositions may be prepared using standard techniques and equipment known to those skilled in the art. The apparatus useful in accordance with the embodiments described herein comprises mixing and heating apparatus well known in the chewing gum manufacturing arts, and therefore the selection of the specific apparatus will be apparent to the artisan.

For instance, the candy or gum may be centre-filled in accordance with the technique disclosed in GB 1432398.

This document describes an apparatus for forming centre-filled chewy composition comprising an orifice means for extruding a rope of candy or gum. The rope is extruded through the orifice around a vent conduit through which the centre-fill is introduced to the hollow centre of the rope. Any gaseous substances, such as air, are vented through a space between the vent conduit and the centre-fill conduit. This precludes the possibility of gaseous bubbles from expanding in the hollow centre of the rope after the top has been extruded through the orifice. Such expansion of gas within the hollow centre would deform the outer perimeter of the top, and also adversely effect the consistency of the centre-fill therein.

Typically the centre-filled rope is fed to into sizer means including at least one, and preferably a plurality of pairs of rollers for progressively decreasing a cross-sectional dimension of the rope. The, or each pair of rollers, may include at least one vertical pair of rollers having vertically aligned axes of rotation and over-lapping lower flange portions. Ramp means are provided for guiding the rope above the flange portions upon entry of the rope between the vertical pairs of rollers. This precludes the possibility of the rope adhering or sticking to one of the lower flange portions and becoming distorted prior to the rope's entry between the rollers.

### Coating Techniques

In some embodiments, in particular where the comestible is a confectionary product, the comestible may be coated. When the confectionary compositions are formed into coated products, the coating may be applied by any method known in the art. The outer coating may be hard, soft or crunchy. Any suitable coating materials known to those skilled in the art may be employed. A number of types of coating are in existence, some of these are described below.

**Gumming (or Glazing):** A technique in which syrups of non-crystallisable and generally non-hygroscopic substances such as gum arabic, modified starches and celluloses and maltodextrins are used. After one or two applications of the syrup to the product to be coated, this technique makes it possible to create a vitreous film forming a barrier to the migration of oxygen, water or fats. Powders of various kinds can also be employed in this process together with these non-crystallisable syrups, so as to bind the water introduced by the syrups. In yet other cases, sugars or polyols which are molten or liquefied by solvents are employed. The hard and brittle vitreous coating is then obtained by cooling or by evaporation of the solvents.

**Soft Coating:** consists in creating a rather flexible and soft coating on the surface of the products. This coating is obtained by repeated applications, on the one hand, of a non-crystallisable syrup such as generally starch hydrolysates and, on the other hand, of a powder, generally of crystallized sucrose. The coating is usually thick. It should be noted that the substance of which the syrup consists is usually other than that of the powder and that no drying is furthermore carried out.

**Hard Coating:** requires the use of a syrup containing crystallisable substances. A hard and crystal-line coating is then obtained by the application of the syrup and evaporation of the water introduced by the latter, by virtue of drying using hot dry air. This cycle must be repeated a very large number of times.

**Smoothing:** consists of one or two applications or charges of a crystallisable syrup which is dilute when compared with that employed in hard coating. The aim is often to apply a finish to the surface appearance of coated products.

**Frosting:** is also aimed at improving the appearance of the product, but also at isolating the latter from atmospheric moisture. This technique resembles a hard coating, in the sense that a crystallisable syrup is employed. The essential difference lies in the fact that the number of cycles carried out is only one, two or three.

**Sanding:** consists in making fine sugar crystals adhere to the surface of the products by moistening them beforehand with rather dry steam or with a hot solution of gum arabic or of maltodextrin. The products are subsequently always dried.

**Wet Crystallization:** is a technique by which a crystalline coating is created on the surface of the products by immersing them for a few hours in a supersaturated syrup of crystallisable substance. The surface of the products is thus embellished by the presence of a coat of microcrystals.

In some embodiments of the invention, the coating per se, or outermost region of the coating, can be formed by lamination, dual or multiple extrusion, or any other process that creates an outermost region.

In panning processes, the coating is initially present as a liquid syrup which contains coating ingredients (for example, as previously described herein), and a solvent such as water. In general, the coating process is carried out in a rotating pan. The comestible core to be coated (for instance a chewy candy or chewing gum) are placed into a rotating pan to form a moving mass. The material or syrup which will eventually form the coating is applied or distributed over the cores. Flavouring agents may be added before, during and after applying the syrup to the cores. Once the coating has dried to form a hard surface, additional syrup additions can be made to produce a plurality of coatings or multiple layers of hard coating.

In a hard coating panning procedure, syrup is typically added to the cores at a temperature range of from 38 -116°C. The syrup temperature is generally from 54-94 °C throughout the process in order to prevent crystallisation. The syrup may be mixed with, sprayed upon, poured over, or added to the core in any way known to those skilled in the art.

In general, a plurality of layers is obtained by applying single coats, allowing the layers to dry, and then repeating the process. The amount of solids added by each coating step depends chiefly on the concentration of the coating syrup. Any number of coats may be applied to the cores but typically no more than 75 - 100 coats will be applied. Those skilled in the art will recognize that in order to obtain a plurality of coated layers, a plurality of pre-measured aliquots of coating syrup may be applied to the cores. It is contemplated, however, that the volume of aliquots of syrup applied to the cores may vary throughout the coating procedure. A typical coating time would be in the range 1 - 3 hours, in some embodiments 1 - 2.5 hours, preferably 2 - 2.5 hours.

Once a coating of syrup is applied to the cores, the cores may be dried in an inert medium. One drying medium comprises air, others include pure gases such as nitrogen. Forced drying air contacts the wet syrup coating in a temperature range of from 20 -100°C. Alternatively, the drying air is in the temperature range of from 30 - 85 °C, in some examples 50 - 80 °C. The relative humidity of the drying air may be less than about 8%, preferably in the range 1 - 6%. The drying air may be passed over and admixed with the syrup coated cores in any way commonly known in the art. Preferably, the drying air is blown over and around or through the bed of the syrup coated cores at a flow rate, for large scale operations, of about 2800 cubic feet per minute. If lower quantities of material are being processed, or if smaller equipment is used, lower flow rates would be used. Typical drying times would be in the region of 1 - 3 hours, preferably 2 - 3 hours for candy cores and preferably 2.5 - 3 hours for chewing gum cores.

Unless otherwise stated each of the integers described in the invention may be used in combination with any other integer as would be understood by the person skilled in the art. This applies equally to the combinations of, for instance, centre-fill solutions, candy cores and outer coatings described in the specific examples below. Further, although all aspects of the invention preferably "comprise" the features described in relation to that aspect, it is specifically envisaged that they may "consist" or "consist essentially" of those features outlined in the claims.

Unless otherwise stated all percentages appearing in the specification are percentages by weight of the composition being described. In addition, unless otherwise stated, all numerical values appearing in this application are to be understood as being modified by the term "about".

### Test Methods

### Determination of Salivation Effect

Quantitative Descriptive Analysis was used to determine the salivation effect of the invention. Seven samples of confectionary were tasted by a panel of 10 -12 trained assessors using the technique of Time-Intensity measurement. The samples differed only in the sweetener component of the product.

This technique involves the testing of the confectionary sample in a sensory booth. The booth contained a computer equipped with software which allowed the panellist to record the perceived level of the mouth-watering sensation with time. Each assessor measured the perceived intensity of salivating mouthfeel at 1 second intervals over a time period of 90 seconds for each sample. This was achieved by initiating a timer within the software as the sample was placed in the mouth. Over the subsequent 90 second period the position of a marker on a linear slide scale was adjusted according to the perceived level of salivation. The data thus obtained was then processed to produce an analysis curve (intensity of effect versus time). The assessment was repeated multiple times (multiple assessors were used and at least three repetitions per assessor) and the result derived from the mean of the values obtained. The parameters determined were as follows:
- Time to maximum salivatory effect, the time taken in seconds for the sample to reach its maximum intensity, a short time being indicative of the desired rapid inception of mouth-watering;
- Intensity of salivation at maximum, the highest point in the curve as recorded by each assessor, a high intensity being generally preferred;
- Area under the analysis curve, the larger this area the greater the total salivation over the 90 second test period; and
- Increase angle, the angle in degrees of ascent from the first recorded value to the maximum intensity, a greater increase angle on the curve illustrates which samples have the greatest increase in salivation per unit time.

### Determination of Cooling Effect

150g of sweetener powder was dissolved in 50ml of distilled water at 37°C with stirring. The drop in temperature was measured after 20 seconds than a further 50ml of distilled water added and the temperature measured after a further 20 seconds. This step was repeated three times to a total volume of water of 200ml. All samples were tested in duplicate and the result derived from the mean of the values obtained. The repeated addition of water was intended to mimic the conditions in the mouth, where more saliva would be produced as chewing of the comestible progresses. As a result, the cooling effect is indicative of the cooling effect that would be observed in the mouth upon dissolution of the sweetener during consumption.

In addition, the Quantitative Descriptive Analysis as described above with regard to the evaluation of salivation was applied to determine the time to maximum cooling effect, the intensity of cooling at maximum, the area under the analysis curve and the increase angle. Cooling was assessed in terms of the temperature decrease in the nose and mouth, at the time of drawing air into the mouth. As with the determination of salivation using this technique, for effective cooling, a larger value is desirable for all parameters except the time to maximum cooling.

### Examples

In order that the present invention may be more readily understood, it will be described further with reference to the figures and to the specific examples hereinafter.
Figure 1 is a graph illustrating the results of a series of tests to determine the salivating effect upon the consumption of each of a range of sweeteners; and
Figures 2a and 2b are photographs of the "lemon-skin" effect obtainable with some galactose panned coatings.

### Example 1: Salivatory Effect of Sweeteners

A series of tests were carried out to determine the mouth-watering effect and cooling effect derived from each of a range of sweeteners. The sweeteners tested were sugar (sucrose), erythritol, isomaltulose, trehalose, galactose, dextrose monohydrate and xylitol. Each sweetener was included in a chewy confectionary composition with the composition shown in Table 1, below. Conventional methods were used to prepare this candy chew.

**Table 1: Test Chew Composition**

| **Component of Chew** | **Wt% of Composition** |
|---|---|
| Test sweetener | 46.0 |
| Glucose | 41.0 |
| Fat | 3.0 |
| Gelatine | 1.5 |
| Acid, colouring, strawberry flavour | 1.5 |
| Water | QS |

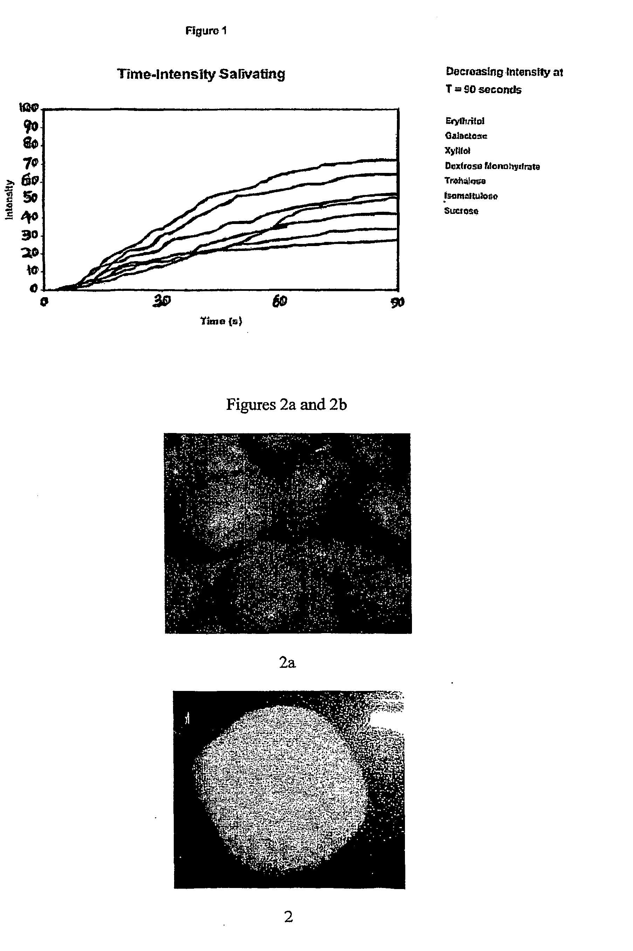

The salivatory tests identified salivation intensity as a function of time and Figure 1 illustrates the results. This figure clearly shows that galactose offers a favourable mouth-watering effect when compared to the other sweeteners and in particular sugar. At the 90 second interval, the mouth-watering effect obtained by galactose is approximately 2.5 times that observed through the use of sugar. As such, the use of galactose in comestible products should be advantageous for the treatment of xerostomia.

Further tests illustrated that not only is the salivatory effect of galactose greater than that of most other sweeteners tested at the time period 90 seconds, but that galactose initialises the salivatory sensation after a shorter chew period, the sensation of salivation reaches its maximum more quickly, has a higher maximum salivatory intensity, a greater total level of perceived salivation over the test period than many other sweeteners tested. Illustrative results are set out in Table 2 below.

**Table 2: Time Intensity Parameters on Salivating Effect^{§}**

| **Sweetener** | **Time to Maximum Salivatory Effect (s)** | **Intensity at Maximum*** | **Area under Curve*** | **Increase Angle (°)** |
|---|---|---|---|---|
| Galactose | 62 | 64.9 | 3561 | 51.5 |
| Dextrose Monohydrate | 76.7 | 52.1 | 2306 | 38 |
| Xylitol | 70.3 | 53.1 | 2874 | 39.3 |
| Trehalose | 66.5 | 43 | 2253 | 35.3 |
| Isomaltulose | 65.7 | 34.3 | 1898 | 31.1 |
| Erythritol | 64.8 | 73 | 4079 | 51.8 |
| Sugar | 58.9 | 28.6 | 1673 | 35.1 |

| | | | | |
|---|---|---|---|---|
| § Mean of at least 13 repetitions * Relative values | | | | |

As can be seen from the results above, none of the other sugars tested show such a consistent benefit in terms of salivatory effect across all the parameters of importance.

### Example 2: Mouth-cooling Effect of Sweeteners

The cooling effect of each of a range of sweeteners was tested using the Chew composition of Example 1, the results are shown in Table 3 below.

**Table 3: Cooling Effect of Sweeteners**

| **Sweetener** | **Magnitude of reduction in temperature at T = 90 seconds (°C)** |
|---|---|
| Dextrose Monohydrate | 14.5 |
| Galactose | 11.0 |
| Maltitol | 16.5 |
| Mannitol | 13.0 |
| Sucrose | 13.5 |
| Xylitol | 26.0 |

It became clear that although offering a small cooling effect, as a result of the associated endothermic heat of hydration, the presence of galactose in a composition does not lead to a product which exhibits a significant cooling effect upon the mouth. This is particularly so when compared to known mouth coolers such as xylitol and erythritol.

Further tests relative to xylitol, erythritol and dextrose monohydrate clearly show that the intensity and overall effect (in terms of area under the cooling curve and increase angle) are lower in galactose than in the other samples assessed.

**Table 4: Time Intensity Parameters on Cooling Effect^{§}**

| **Sweetener** | **Time to Maximum Salivatory Effect (s)** | **Intensity at Maximum*** | **Area under Curve*** | **Increase Angle (°)** |
|---|---|---|---|---|
| Galactose | 57.1 | 14.6 | 725 | 39.3 |
| Dextrose Monohydrate | 81.9 | 24 | 663 | 36.5 |
| Xylitol | 64.5 | 44.7 | 2457 | 46 |
| Erythritol | 63.5 | 59.9 | 3431 | 81.5 |

| | | | | |
|---|---|---|---|---|
| § Mean of at least 10 repetitions * Relative values | | | | |

As can be seen from the results above, the cooling effect of galactose is not particularly marked.

This combination of effective induction of salivation without cooling offers a new and interesting flavour combination, previously not available for the treatment of xerostomia. Further, although erythritol provides a greater mouth-watering effect in these tests, this is combined with a strong cooling sensation. Such cooling sensations can mask flavourings, limiting the choice of flavour which may be utilised in the comestible product. However, the use of galactose, with its near comparable mouth-watering properties is possible with a wide range of flavourings, some of which would be masked by, or simply unpalatable with, a sweetener which also exhibits strong cooling effects.

### Example 3: Effect of Galactose Levels upon Salivatory Effect

Tests were carried out to determine the effect of increasing the galactose level in a chew composition. The test composition had the following formulation:

**Table 5: Soft Chew**

| **Component** | **Polydextrose: Galactose Ratio (wt% of composition)** | |
|---|---|---|
| | **50:50** | **40:60** |
| Polydextrose | 44.02 | 39.02 |
| Sucrose ester SE 5S | 0.20 | 0.20 |
| Copra | 3.09 | 3.09 |
| Gelatine | 2.04 | 2.04 |
| Galactose | 50.22 | 55.22 |
| Colouring | 0.01 | 0.01 |
| Strawberry flavour | 0.42 | 0.42 |
| **Evaluation with Regard to Mouth-watering** | Mouth-watering comes later in chew. | Strong mouth-watering |

The above results illustrate that, in general, the higher the level of galactose in a product, the more distinct the mouth-watering sensation is.

### Example 4: Vanilla Flavoured Soft Chew Confectionary Composition

A centre-fill, hard-coated, soft chew was prepared having the following composition:

**Table 6: Soft Chew**

| **Component of Chew** | **Wt% of zone** |
|---|---|
| *Centre-fill:* | |
| Thin galactose (particle size less than 30 µm) | 98.5 |
| Encapsulated malic acid | 0.5 |
| Dry flavouring | 2g/kg |
| Water | 1.0 |
| *Chew:* | |
| Galactose | 46.0 |
| Glucose | 41.0 |
| Fat | 3.0 |
| Gelatine | 1.5 |
| Colouring, vanilla flavour | 1.5 |
| Water | QS |
| *Coating: (Brix 72)* | |
| Galactose | 85.0 |
| Polydextrose | 15.0 |

The chew composition was found to have a pleasant soft texture and the coating was crispy and smooth.

**Method:** The centre-fill and chew compositions were prepared using conventional techniques. The coating was prepared by combining the galactose and polydextrose with warming then transferring the mixture to a small drum (D&F 5 - 6 kg capacity). The solution was spread by hand and the panned comestible dried at 80°C. The rolling time was 2 hours and 25 minutes, the drying time 2 hours and 30 minutes.

### Example 5: Lemon Flavoured Soft Chew Confectionary Composition (Centre-fill)

A further chew was prepared in accordance with Example 4 but with a modified centre-fill composition comprising, by weight of the centre-fill composition, 97 wt% galactose (particle size less than 30 µm), 3 wt% encapsulated citric acid and 2g/kg lemon juice flavour. The chew flavour was also altered to a lemon flavour to correspond with that of the centre-fill composition. The combination of the lemon and galactose was found to offer a particularly pleasant flavour sensation.

### Example 6: Effervescent Soft Chew Confectionary Composition (Centre-fill)

A chew was prepared in accordance with Example 4 but with a modified centre-fill composition comprising, by weight of the centre-fill composition, 10 wt% citric acid, 10 wt% sodium bicarbonate and 80 wt% galactose.

### Example 7: Lemon Flavoured Soft Chew Confectionary Composition (Coating)

A chew was prepared in accordance with Example 4 but with an alternative hard coating comprising a soft-panning inner coating and a hard-panning outer coating. The hard panning composition comprised, by weight of the coating composition, 56.5 wt% galactose, 11.0 wt% mannitol, 2.5 wt% gelatine, 1.3 wt% citric acid, 0.7 wt% colouring and flavouring and 28.0 wt% water. The soft panning composition was a 10 wt% solution of the hard panning solution. The chew flavour was also altered to a lemon flavour to correspond with that of the hard coating composition.

### Example 8: Vanilla Flavoured Soft Chew Confectionary Composition

A vanilla flavoured soft chew was prepared using the method below to the formulation outlined in Table 7 below.

**Table 7: Soft Chew**

| **Component** | **Wt% of Composition** |
|---|---|
| Polydextrose | 44.09 |
| Sucrose ester SE 5S | 0.20 |
| Copra | 3.06 |
| Gelatine | 1.52 |
| Galactose | 49.51 |
| Encapsulated malic acid | 0.64 |
| Vanilla flavour | 0.98 |

**Method:** The polydextrose was added to water which had been heated to 90 °C with stirring. The temperature was increased to 110°C and maintained until complete dissolution of the polydextrose. The fat and emulsifier were then added with stirring under high shear and the temperature raised to 125.5 °C to reduce the moisture level of the mixture to 9.9 wt%. This mixture was then transferred to a Rayneri Z-arm mixer and the gelatine, and galactose added with stirring at a temperature of 125.5 °C. Once the moisture level of the resulting mixture had been reduced to 6.7 wt%, the malic acid and flavourings were added and stirred until mixed. The final product contained 7.0 wt% water.

This produced a good firm dough when cool, with good cohesiveness and texture (not too sticky). This formulation was found to be particularly suitable for use in smaller sized chews.

### Example 9: Lime Flavoured Soft Chew Confectionary Composition

A lime flavoured soft chew having the following composition was prepared.

**Table 8: Soft Chew**

| **Component** | **Wt% of Composition** |
|---|---|
| Polydextrose | 43.76 |
| Sucrose ester SE 5S | 0.20 |
| Copra | 3.04 |
| Gelatine | 1.50 |
| Galactose | 50.14 |
| Encapsulated malic acid | 1.15 |
| Colouring | 0.01 |
| Lime flavour | 0.21 |

The method used for the preparation of the chew was as for Example 8 and produced a chew with good processing properties and a pleasant flavour which was well suited to coating.

### Example 10: Mint Flavoured Soft Chew Confectionary Composition

The composition of this example was the same as that of Example 9 except that it was mint flavoured. The resulting chew was found to have good processing properties and had a texture suitable for use without the need for an external coating.

### Example 11: Centre-fill Chewing Gum Composition

A centre-fill, chewing gum pellet was prepared using conventional techniques having the following composition:

**Table 9: Chewing Gum Composition**

| **Component of Chewing Gum** | **Wt% of zone** |
|---|---|
| *Centre-fill: 15 wt% of composition* | |
| Thin galactose (particle size less than 30 µm) | 97.0 |
| Encapsulated citric acid | 3.0 |
| Dry flavouring | 2g/kg |
| *Gum: 85 wt% of composition* | |
| Galactose | 46.0 |
| Polydextrose | 17.0 |
| Gum Base | 33.0 |
| Others | 4.0 |

The gum was found to have a pleasant soft texture and offer a mouth-watering effect.

### Example 12: Lemon Skin Galactose Coating

A galactose coating was prepared in accordance with the formulation below for use in coating lemon or other citrus flavour chews.

**Table 10: Lemon Skin Coating**

| **Component of Coating** | **Wt% of Composition** |
|---|---|
| Galactose | 86.37 |
| Sodium citrate | 2.67 |
| Citric acid | 2.78 |
| Polydextrose | 5.11 |
| Gelatine | 3.08 |

**Method:** The components of the coating composition were mixed in a pan with warming. Once homogenised, the coating was transferred to a small coating drum (D&F 5 - 6 kg capacity) and the solution spread by hand. The panned comestible, in this specific example a lemon flavoured galactose chew in accordance with the invention, was dried in an air conditioned environment at 76°C. The drying time was approximately 2.5 - 3 hours.

The coating was crisp and smooth with a distinctive "lemon skin" appearance, as shown in Figures 2a and 2b.

It should be appreciated that the compositions and methods of the invention are capable of being incorporated in the form of a variety of embodiments, only a few of which have been illustrated and described above.

## Claims

1. A comestible product selected from candy and chewing gum comprising more than one zone, said zones being selected from a centre-fill region, a candy or chewing gum core and an outer coating, wherein galactose is the primary sweetener in at least one zone of the comestible product, said zone comprising 40 - 100 wt% of the total sweetener of that zone galactose; and wherein galactose comprises from 40 - 95 wt% of the comestible product; and whereby the galactose has a particle size less than about 30 µm.

2. A comestible product according to claim 1 wherein galactose is present in the at least one zone of the comestible in an amount greater than the total amount of the other sweeteners present in that zone.

3. A comestible product according to any preceding claim wherein galactose is present in the comestible product in an amount greater than the total amount of other sweeteners in the comestible.

4. A comestible product according to any preceding claim wherein the comestible includes a food-grade acid or salt thereof.

5. A comestible product according to claim 1 wherein galactose is the primary sweetener in more than one zone of the comestible product.

6. A comestible product according to claim 1 wherein at least one zone does not contain galactose as the primary sweetener.

7. A comestible product according to claim 6 wherein at least one zone is free from galactose.

8. A comestible product according to claim 1 comprising at least a candy core and a second zone selected from a centre-fill region and an outer coating.

9. A comestible product according to claim 8 wherein galactose is the primary sweetener in the second zone.

10. A comestible product according to claim 8 or 9 wherein the second zone comprises 60 - 98 wt% galactose.

11. A comestible product according to any preceding claim wherein at least one zone is free from a food-grade acid or salt thereof.

12. A comestible product according to any of claims 8 to 11 wherein the second zone is a hard coating and wherein the comestible core is pre-coated prior to addition of the outer coating.

13. A comestible product according to any of claims 8 to 12 wherein the second zone is a hard coating which includes a crystallisation retarding component selected from a gum, polydextrose, sodium citrate, gelatine, mannitol, and/or combinations thereof.

14. A comestible product according to claim 13 wherein the crystallisation retarding component is selected from a gum present in the range 1 - 10 wt% of the coating composition; and polydextrose and/or mannitol present in the range 10 - 20 wt% of the coating composition.

15. A comestible product according to any preceding claim which is free of a cooling agent.

16. A method of improving the mouth-watering effect of a comestible comprising incorporating galactose into the comestible.

17. A method according to claim 16 wherein the comestible additionally comprises a food-grade acid.

18. A method of preparing a comestible according to any of claims 1 to 15 comprising the incorporation of galactose into the comestible.

19. The use of a comestible according to any of claims 1 - 15 in the manufacture of a medicament for the treatment of xerostomia.

## Patentansprüche

1. Essbares Produkt ausgewählt aus Süßigkeiten und Kaugummi mit mehr als einer Zone, wobei die Zonen ausgewählt sind aus einem Zentralfüflbereich, einem Süßigkeiten- oder Kaugummikern und einer äußeren Beschichtung, wobei Galactose der Hauptsüßstoff in zumindest einer Zone des essbaren Produktes ist, und die Zone 40-100 Gewichtsprozent des Gesamtsüßstoffes jener Galactosezone enthält; und wobei Galactose mit 40-95 Gewichtsprozent in dem essbaren Produkt enthalten ist; und wodurch die Galactose eine Teilchengröße von weniger als etwa 30 µm aufweist.

2. Essbares Produkt nach Anspruch 1, wobei Galactose in der zumindest einen Zone des Lebensmittels in einer Menge vorliegt, die größer ist als die Gesamtmenge der anderen Süßstoffe, die in jener Zone vorliegen.

3. Essbares Produkt nach einem vorstehenden Anspruch, wobei Galactose in dem essbaren Produkt in einer Menge vorliegt, die größer ist als die Gesamtmenge der anderen Süßstoffe in dem Lebensmittel.

4. Essbares Produkt nach einem vorstehenden Anspruch, wobei das Lebensmittel eine Säure in Lebensmittelqualität oder ein Salz davon enthält.

5. Essbares Produkt nach Anspruch 1, wobei Galactose der Hauptsüßstoff in mehr als einer Zone des essbaren Produktes ist.

6. Essbares Produkt nach Anspruch 1, wobei zumindest eine Zone Galactose nicht als den Hauptsüßstoff enthält.

7. Essbares Produkt nach Anspruch 6, wobei zumindest eine Zone frei von Galactose ist.

8. Essbares Produkt nach Anspruch 1, mit zumindest einem Süßigkeitskern und einer zweiten Zone ausgewählt aus einem Zentralfüllbereich und einer äußeren Beschichtung.

9. Essbares Produkt nach Anspruch 8, wobei Galactose der Hauptsüßstoff in der zweiten Zone ist.

10. Essbares Produkt nach Anspruch 8 oder 9, wobei die zweite Zone 60-98 Gewichtsprozent Galactose enhält.

11. Essbares Produkt nach einem vorstehenden Anspruch, wobei zumindest eine Zone von einer Säure in Lebensmittelqualität oder einem Salz davon frei ist.

12. Essbares Produkt nach einem der Ansprüche 8 bis 11, wobei die zweite Zone eine harte Beschichtung ist, und wobei der essbare Kern vor der Zugabe der äußeren Beschichtung vorbeschichtet ist.

13. Essbares Produkt nach einem der Ansprüche 8 bis 12, wobei die zweite Zone eine harte Beschichtung ist, welche eine kristallisationsverzögernde Komponente enthält, die ausgewählt ist aus einem Gummi, Polydextrose, Natriumcitrat, Gelatine, Mannitol und/oder Kombinationen davon.

14. Essbares Produkt nach Anspruch 13, wobei die kristallisationsverzögernde Komponente ausgewählt ist aus einem Gummi, der in dem Bereich von 1-10 Gewichtsprozent der Beschichtungszusammensetzung vorliegt; und Polydextrose und/oder Mannitol, die in dem Bereich von 10-20 Gewichtsprozent der Beschichtungszusammensetzung vorliegen.

15. Essbares Produkt nach einem vorstehenden Anspruch, das frei von einem Fühlmittel ist.

16. Verfahren zum Verbessern der mundwässernden Wirkung eines Lebensmittels mit Aufnahme von Galactose in das Lebensmittel.

17. Verfahren nach Anspruch 16, wobei das Lebensmittel zusätzlich eine Säure in L.ebensmittelqualität enthält.

18. Verfahren zum Zubereiten eines Lebensmittels nach einem der Ansprüche 1 bis 15 mit der Aufnahme von Galactose in das Lebensmittel.

19. Verwendung eines Lebensmittels nach einem der Ansprüche 1-15 bei der Herstellung eines Medikamentes für die Behandlung von Xerostomie.

## Revendications

1. Produit comestible choisi parmi un bonbon et un chewing-gum comprenant plus d'une zone, lesdites zones étant choisies parmi une région de remplissage centrale , un coeur de bonbon ou de chewing-gum et une enveloppe externe, dans lequel le galactose est l'édulcorant principal dans au moins une zone du produit comestible, ladite zone comprenant un niveau de galactose représentant 40 à 100 % en poids de l'édulcorant total de cette zone ; et dans lequel le galactose représente de 40 à 95 % en poids du produit comestible ; et où le galactose a une taille des particules inférieure à environ 30 µm.

2. Produit comestible selon la revendication 1 dans lequel le galactose est présent dans la au moins une zone du produit comestible en une quantité supérieure à la quantité totale des autres édulcorants présents dans cette zone.

3. Produit comestible selon l'une quelconque des revendications précédentes dans lequel le galactose est présent dans le produit comestible en une quantité supérieure à la quantité totale des autres édulcorants du produit comestible.

4. Produit comestible selon l'une quelconque des revendications précédentes dans lequel le produit comestible comprend un acide de qualité alimentaire ou un sel de celui-ci.

5. Produit comestible selon la revendication 1 dans lequel le galactose est l'édulcorant principal dans plus d'une zone du produit comestible.

6. Produit comestible selon la revendication 1 dans lequel au moins une zone ne contient pas de galactose en tant qu'édulcorant principal.

7. Produit comestible selon la revendication 6 dans lequel au moins une zone est exempte de galactose.

8. Produit comestible selon la revendication 1 comprenant au moins un coeur de bonbon et une seconde zone choisie parmi une région de remplissage central et une enveloppe externe.

9. Produit comestible selon la revendication 8 dans lequel le galactose est l'édulcorant principal dans la seconde zone.

10. Produit comestible selon la revendication 8 ou 9 dans lequel la seconde zone comprend de 60 à 98 % en poids de galactose.

11. Produit comestible selon l'une quelconque des revendications précédentes dans lequel au moins une zone est exempte d'acide de qualité alimentaire ou de sel de celui-ci.

12. Produit comestible selon l'une quelconque des revendications 8 à 11 dans lequel la seconde zone est une enveloppe dure et dans lequel le coeur comestible est pré-enrobé avant addition de l'enveloppe externe.

13. Produit comestible selon l'une quelconque des revendications 8 à 12 dans lequel la seconde zone est une enveloppe dure qui comprend un composant retardant la cristallisation choisi parmi une gomme, de la polydextrose, du citrate de sodium, de la gélatine, du mannitol et/ou leurs combinaisons.

14. Produit comestible selon la revendication 13 dans lequel le composant retardant la cristallisation est choisi parmi une gomme présente dans l'intervalle de 1 à 10 % en poids de la composition de l'enveloppe ; et de la polydextrose et/ou du mannitol présents dans l'intervalle de 10 à 20 % en poids de la composition de l'enveloppe.

15. Produit comestible selon l'une quelconque des revendications précédentes qui est exempt d'agent rafraîchissant.

16. Procédé d'amélioration de l'effet d'hydratation buccale d'un produit comestible comprenant l'incorporation de galactose dans le produit comestible.

17. Procédé selon la revendication 16 dans lequel le produit comestible comprend en outre un acide de qualité alimentaire.

18. Procédé de préparation d'un produit comestible selon l'une quelconque des revendications 1 à 15 comprenant l'incorporation de galactose dans le produit comestible.

19. Utilisation d'un produit comestible selon l'une quelconque des revendications 1 à 15 dans la fabrication d'un médicament pour le traitement de la xérostomie.
